# EUROPEAN PATENT APPLICATION

(11) **EP 4 667 044 A2**
(43) Date of publication of application: **24.12.2025**
(21) Application number: 25214722.8
(22) Date of filing: 08.11.2019
(51) Int. Cl.: A61N 1/32

(54) **NERVE REGENERATION THERAPY SYSTEM**

(30) Priority: 08.11.2018 US 201862757444 P
(62) Divisional of application: 19881365.1
(71) Applicant: Checkpoint Surgical, Inc., Cleveland, Ohio 44122 (US)
(72) Inventor: SCANLAN, Kevin, Cleveland (US); WALKER, Eric R., Berea (US); COSENTINO, Leonard M., Hudson (US); LEWIS, Derek, Cleveland (US); COTTRILL, Ben, Cleveland (US)
(74) Representative: Zacco Sweden AB

(57) **Abstract**

An electrical stimulation system includes a stimulation device and a container. The stimulation device may be sealed within the container. A port in the container allows the stimulation device to be coupled with a lead. The stimulation device may be used before, during, and after subcutaneous surgery to apply electrical stimulation for nerve regeneration.

## Description

### CROSS-REFERENCE TO RELATED APPLICATION

This application claims priority to U.S. Provisional Patent Application No. 62/757,444 entitled "Nerve Regeneration Therapy Stem and Method," filed on November 8, 2018, which is incorporated herein by reference in its entirety.

### FIELD OF THE INVENTION

The invention relates generally to nerve stimulation, and more particularly to systems and methods for accelerating or enhancing regrowth or recovery of injured or potentially injured nerves before, during, after, or independent of surgical procedures.

### BACKGROUND OF THE INVENTION

Nerve injuries present clinicians with significant challenges in determining the proper course of treatment to restore impaired motor and or sensory function. Ultimately, the severity of the nerve injury and time post injury has the greatest influence on the treatment plan and potential for success. In many cases, surgical intervention may be needed to increase the likelihood that control of muscle function or sensation can be regained. Surgical treatment of nerve injuries typically does not provide immediate restoration of function, as nerve fibers must grow from the point of intervention or repair to the target muscle. Nerve fibers grow at a rate of about 1-3 mm/day, and thus recovery takes a significant amount of time.

Despite advancements in surgical technique and medical device technology, the rate of growth and organization of fiber growth direction remains a significant factor limiting functional outcomes.

It may be desirable to provide a method for delivering a period of electrical stimulation instead of a surgical procedure, prior to a procedure, and possibly in the operating room before the procedure, to improve functional outcome. It may also be desirable to provide stimulation to an injured nerve either in a medical office or outside of an office without the need for a follow up surgical procedure or other procedure. Furthermore, it may be desirable to be able to initiate a period of stimulation prior to a procedure, then in the operating room and continuing into a post-operative setting. It may alternatively or additionally be desirable to initiate stimulation during surgery and/or after surgery. Moreover, stimulation may be initiated in an office or an operating room and may continue while the patient moves between rooms. Additionally, it may be desirable to deliver repeated periods of stimulation during recovery or as part of a therapy of nerve recovery, without the need to replace electrode(s) before each application.

### SUMMARY OF THE INVENTION

The following presents a summary of this disclosure to provide a basic understanding of some aspects. This summary is intended to neither identify key or critical elements nor define any limitations of embodiments or claims. Furthermore, this summary may provide a simplified overview of some aspects that may be described in greater detail in other portions of this disclosure.

An electrical stimulation system includes a stimulation device comprising a housing; control circuitry operatively generating a stimulation signal, wherein the control circuitry is disposed within the housing; and a container housing the stimulation device, the container comprising at least one port, and wherein the container may be (or may not be) hermetically sealed.

A method for stimulating tissue includes providing a stimulation device; placing a lead within range of a target tissue region, wherein the lead is operatively attached to the stimulation device; applying a stimulation signal to the target tissue region with the lead and the stimulation device; terminating the applying stimulation signal; and performing a subcutaneous surgery after terminating the applying stimulation signal.

Also disclosed is a method for stimulating tissue, comprising providing a stimulation device, placing the stimulation device within a container comprising at least one port, and wherein the container may be or may not be hermetically sealed, placing a lead within range of a target tissue region, wherein the lead is operatively attached to the stimulation device through the at least one port, and applying a stimulation signal to the target tissue region with the lead and the stimulation device for treatment of the target tissue region.

The following description and the drawings disclose various illustrative aspects. Some improvements and novel aspects may be expressly identified, while others may be apparent from the description and drawings.

### BRIEF DESCRIPTION OF THE DRAWINGS

The accompanying drawings illustrate various systems, apparatuses, devices and methods, in which like reference characters refer to like parts throughout.
FIG. 1 illustrates a partial cross-sectional view of a stimulation device, in accordance with various disclosed aspects;
FIG. 2 illustrates a side view of the stimulation device of FIG. 1, in accordance with various disclosed aspects;
FIG. 3 illustrates a percutaneous electrode, in accordance with various disclosed aspects;
FIG. 4A illustrates another percutaneous electrode, in accordance with various disclosed aspects;
FIG. 4B illustrates another percutaneous electrode, in accordance with various disclosed aspects;
FIG. 5 illustrates an adaptor comprising a percutaneous electrode, in accordance with various disclosed aspects;
FIG. 6 illustrates an adaptor comprising two connectors, in accordance with various disclosed aspects;
FIG. 7 illustrates the stimulation device of FIG. 1 coupled with an adaptor, in accordance with various disclosed aspects;
FIG. 8 illustrates a top view of a container for receiving a stimulation device, in accordance with various disclosed aspects;
FIG. 9 illustrates a top view of another container for receiving a stimulation device, in accordance with various disclosed aspects;
FIG. 10 illustrates a back view of the container of FIG. 9, in accordance with various disclosed aspects;
FIG. 11 illustrates a system comprising the container of FIG. 8, the stimulation device of FIG. 1, and electrode connections, in accordance with various disclosed aspects;
FIG. 12 illustrates a perspective view of a splitter device, in accordance with various disclosed aspects;
FIG. 13 illustrates a partial cross-sectional, perspective view of a splitter device, in accordance with various disclosed aspects;
FIG. 14 illustrates a perspective view of the splitter device of FIG. 12 coupled with percutaneous leads, in accordance with various disclosed aspects;
FIG. 15 illustrates a perspective view of another stimulation device, in accordance with various disclosed aspects;
FIG. 16 illustrates an embodiment of the stimulation device in FIG. 15 without a belt, in accordance with various disclosed aspects;
FIG. 17 illustrates a back view of the stimulation device of FIG. 16, in accordance with various disclosed aspects; and
FIG. 18 illustrates an embodiment of the stimulation device in FIG. 15 with one or more percutaneous leads and a return electrode, in accordance with various disclosed aspects.

The invention may be embodied in several forms without departing from its spirit or essential characteristics. The scope of the invention is defined in the appended claims, rather than in the specific description preceding them. All embodiments that fall within the meaning and range of equivalency of the claims are therefore intended to be embraced by the claims.

### DESCRIPTION OF PREFERRED EMBODIMENTS

Reference will now be made in detail to exemplary embodiments of the present invention, examples of which are illustrated in the accompanying drawings. It is to be understood that other embodiments may be utilized, and structural and functional changes may be made without departing from the respective scope of the invention. Moreover, features of the various embodiments may be combined or altered without departing from the scope of the invention. As such, the following description is presented by way of illustration only and should not limit in any way the various alternatives and modifications that may be made to the illustrated embodiments and still be within the spirit and scope of the invention.

As used herein, the words "example" and "exemplary" mean an instance, or illustration. The words "example" or "exemplary" do not indicate a key or preferred aspect or embodiment. The word "or" is intended to be inclusive rather an exclusive, unless context suggests otherwise. As an example, the phrase "A employs B or C," includes any inclusive permutation (e.g., A employs B; A employs C; or A employs both B and C). As another matter, the articles "a" and "an" are generally intended to mean "one or more" unless context suggests otherwise.

It is noted that the various embodiments described herein may include other components and/or functionality. It is further noted that while various embodiments refer to a stimulator or stimulation device, various other systems may be utilized in view of embodiments described herein. For example, embodiments may be utilized in a variety of surgical procedures, other medical procedures or as a stand-alone procedure. As such, embodiments may refer to a particular surgical procedure for purposes of explanation. It is noted that aspects of embodiments, however, may be utilized for various other procedures or as a stand-alone procedure.

This disclosure generally relates to systems and methods that may improve nerve healing, including nerve regeneration, Wallerian degeneration, axonal regeneration, or neuroregeneration of tissue via electrical stimulation to increase the speed or amount of nerve growth. The terms "nerve" or "nerve tissue" generally refer to any portion of a nerve including, but not limited to, axons, axon terminals, somas, dendrites, or the like, unless context suggests otherwise. Moreover, aspects disclosed herein may be applicable to nerve tissue throughout a body, whether peripheral nervous tissue or otherwise. Further, while embodiments may reference a surgeon performing a particular action(s), it is noted that other clinicians or users, automated machines, or the like may perform such actions.

In an embodiment, an electrical stimulation system may comprise a stimulation device that comprises a housing; control circuitry operatively generating a stimulation signal, wherein the control circuitry is disposed within the housing; a container housing the stimulation device, the container comprising at least one port, and wherein the container is sealed, wherein the container prevents contamination of the stimulation device such that the stimulation device may be used during at least two occasions selected from before subcutaneous surgery, during subcutaneous surgery, or after subcutaneous surgery.

The electrical stimulation may also include the following in any combination:
- wherein the port operatively coupels a lead to the stimulation device;
- wherein the stimulation device operatively generates a first stimulation signal for application to a target tissue region before the subcutaneous surgery and a second stimulation signal after surgery for stimulation of the target tissue region after the subcutaneous surgery;
- wherein the stimulation device operatively generates a third stimulation signal for stimulation of the target tissue region during the subcutaneous surgery;
- wherein the stimulation device terminates generation of the first stimulation signal before the subcutaneous surgery begins and does not generate another stimulation signal until after the subcutaneous surgery ends;
- wherein the stimulation device generates the first stimulation signal between generally ten minutes and generally 15 minutes;
- wherein the stimulation device generates the second stimulation signal between generally ten minutes and generally 15 minutes;
- wherein the stimulation device generates the third stimulation signal between generally ten minutes and generally 15 minutes; and/or
- wherein the first stimulation signal comprises different attributes as the second stimulation signal.
- wherein the stimulation device further comprises a pulse counter operatively counting the number of pulses generated by the control circuitry.
- wherein the stimulation device further comprises a timer operatively measuring passage of time during which the control circuitry is generating a stimulation signal.
- wherein the stimulation device further comprises an action potential sensors operatively sensing invoked action potential through the target tissue region.

In an embodiment, a method for stimulating tissue may comprise providing a stimulation device and a container housing the stimulation device; placing a lead within range of a target tissue region, wherein the lead is operatively attached to the stimulation device; applying a first stimulation signal to the target tissue region with the lead and the stimulation device; terminating the applying the first stimulation signal; performing, after terminating the application of the first stimulation signal, a subcutaneous surgery; and applying, after terminating the application of the first stimulation signal, a second stimulation signal to the target tissue region.

The forgoing method may also comprise any of the foregoing in any combination:
- applying the second stimulation signal after the subcutaneous surgery;
- applying a third stimulation signal during the subcutaneous surgery;
- applying the second stimulation signal during the subcutaneous surgery;
- applying the first stimulation signal comprises applying the first stimulation signal for at least generally ten minutes; and/or.
- applying the second stimulation signal comprises applying the second stimulation signal for at least generally ten minutes.

An embodiment of an electrical stimulation system may comprise a stimulation device that comprises a housing; control circuitry operatively generating a stimulation signal, wherein the control circuitry is disposed within the housing; wherein the stimulation device operatively generates a first stimulation signal for application to a target tissue region to promote a healing process of a first mechanism and wherein the stimulation device operatively generates a second stimulation signal for stimulation of the target tissue region to promote a healing process of a second mechanism.

The electrical stimulation system may including any of the foregoing in any combination:
- wherein the first stimulation signal is applied for a first dosing period, the first dosing period being between generally ten minutes and generally 15 minutes;
- wherein the first stimulation signal is applied for a plurality of dosing periods, wherein each of the dosing periods are for an equivalent amount of time;
- wherein the first stimulation signal is applied for a plurality of dosing periods, wherein each of the dosing periods are for a different amount of time; and/or
- The electrical stimulation system of claim 16, wherein the second stimulation signal is applied between generally ten minutes and generally 15 minutes.

It is noted that described systems and methods may be utilized in combination with various systems and methods for safeguarding against nerve, muscle, and tendon injury during surgical procedures, other procedures or as a stand-alone procedure or confirming the identity and/or location of nerves, muscles, and tendons and evaluating their function or the function of muscles enervated by those nerves. The systems and methods are particularly well suited for assisting in nerve regeneration via a device that may also be utilized by a surgeon or clinician in identification of nerves and muscles in order to assure nerve and muscle integrity during medical procedures using medical devices such as stimulation monitors, cutting, drilling, and screwing devices, pilot augers, and fixation devices, including without limitation, the system disclosed in U.S. Patent No. 10470678 which is incorporated herein by reference. It is noted, however, that described systems and methods may utilize other devices that are not utilized during surgical procedures. Moreover, various disclosed aspects may be utilized independent of such systems and methods.

Embodiments described herein provide stimulation of a target nerve or nerves as an alternative to or during a surgical procedure or prior to a surgical or other medical procedure, including without limitation, peri-operatively. This pre-procedure, in-procedure or stand-alone stimulation may result in more efficient recovery in comparison to conducting procedures without pre-procedure stimulation or any stimulation. Efficient recovery may include reduced time for nerve regeneration, reduced need for nerve regeneration, improved nerve functionality post operation, reduced need (or no need) for surgical intervention, or the like. Additionally or alternatively, embodiments may utilize successive stimulation delivered as treatment without surgery, during surgery, prior to repair or treatment of the nerve injury, and/or following repair or treatment of the nerve injury and/or continue post-surgery.

In an example, a surgeon, clinician or other medical professional may apply one or more leads to a user. The leads may be percutaneous, trans-cutaneous, surface electrodes, or leads directly attached to the nerve. The leads may be placed in a region suspected of nerve damage or diminished function, a region of possible nerve damage or diminished function, and/or a region where an operation is to be performed or is being performed. For instance, a procedure may be planned that requires operation at or near a nerve. This may put the nerve at risk for damage, temporary or permanent diminished function, or rehabilitation after the procedure. Described embodiments stimulate the nerve prior to the surgical procedure. Stimulation prior to the procedure may reduce recovery or rehabilitation time after the procedure, may reduce or prevent diminished function, or the like. Moreover, the surgeon may apply additional stimulation during or after the procedure in combination with or as an alternative to the stimulation prior to the procedure. The additional stimulation may, in combination with the pre-procedure stimulation, provide benefits to nerve rehabilitation. In addition or in the alternative, the clinician may apply the stimulation without performing a procedure in an effort to aid in nerve healing or regeneration.

In various embodiments described herein, systems and methods provide for stimulation of a target nerve or nerves as a treatment to the target nerve or nerves for the purpose of nerve regeneration and/or healing of such nerve or nerves. Accordingly, while some examples may refer to post and pre-operative stimulation, various embodiments may include stimulation without surgical operations or in replacement of surgical intervention. Still further, such stimulation may be provided during the applicable surgery. It is noted that similar or different stimulation parameters (e.g., stimulation patterns, intensity, duration, frequency, single does, repeated dosing, etc.) may be applied to stimulation without surgery, or to stimulation during post and pre-operative surgery. Further, the stimulation parameters may or may not be related to the type of stimulation being applied, i.e., if it is part of surgery, pre-operative, during surgery, post-operative or with no surgery. In other embodiments, the stimulation parameters may all be the same regardless of the type of stimulation being applied.

Described procedures may result in more efficient recovery or regeneration of damaged or potentially damaged nerves. The stimulation may be provided as a treatment itself, independent of any surgical procedures, or may be the entire surgical procedure. It is noted that stimulation may be conducted via percutaneous delivery of stimulation, transcutaneous delivery of stimulation, transdermal delivery (e.g., implanted electrode and surface coil for power delivery and communication), or a combination thereof.

By way of a non-limiting example in a transdermal delivery, an electrode may be implanted and may be wirelessly and operatively coupled with a surface coil or may be directly and operatively attached with the surface coil for power delivery and communication so that the electrode may apply the appropriate stimulation. The electrode may be positioned in any appropriate manner, such as through a needle, through surgical intervention or any other appropriate method.

In an example, stimulation of target nerve tissue for nerve regeneration may be applied as treatment in place of or as an alternative to surgery. For instance, targeted electrical stimulation may be applied to the applicable nerve for carpal tunnel treatment (although that is merely exemplary and the electrical stimulation may be applied to any kind of nerve injury, damage or otherwise for preventative measure). The treatment may include immobilization and stimulation therapy to avoid surgery if possible. In another example, stimulation may be applied after an injury as the primary treatment. A surgeon or other professional may place an electrode and may stimulate nerve tissue (or nerve fibers), or surrounding tissues that support nerve healing (Schwan cells, macrophage cells) to accelerate regeneration and healing when surgical intervention is not needed, such as to treat muscle weakness, loss of sensation after traumatic or prolonged injury, or other issues relating to the nerve or surrounding tissue. In some situations, this treatment may be successful enough for a patient such that surgical intervention can be avoided. The stimulation therapy may accelerate nerve regeneration, which may accelerate healing enough that surgery is not needed.

In at least some embodiments, a surgeon or clinician may utilize a handheld stimulation device to generate a stimulation signal at sufficiently high levels for the purposes of locating, stimulating, and evaluating nerve or muscle, or both nerve and muscle integrity in numerous medical procedures. This may also include, but is not limited to, evaluating proximity to a targeted tissue region, evaluating proximity to a nerve or to identify nerve tissue, evaluating nerve integrity (i.e., following a traumatic or repetitive motion injury) to determine if a repair may be needed, evaluating muscle contraction to determine whether or not the muscle is innervated and/or whether the muscle is intact and/or whether the muscle is severed, identifying specific nerve branches or fascicles for repair or transfer, and evaluating muscle and tendon length and function following a repair or tendon transfer prior to completing a surgical procedure.

Before the procedure or for treatment without a separate procedure, a surgeon may place an electrode or lead on or near the nerve to be stimulated and/or proximal to the site of injury, potential injury, or repair. The electrode may be percutaneous or non-percutaneous (e.g., surface electrode or implanted electrode). In some embodiments, the electrode may be percutaneously positioned while a component transmits energy to the percutaneously positioned electrode through tissue. It is noted that the transmitting component may be positioned on the skin at an appropriate location, i.e., it may be an external electrical stimulator (or pulse generator). In an aspect, a percutaneous lead may be taped or otherwise held in place on a patient's skin. This may allow for easy removal after prolonged stimulation. One exemplary embodiment of such comprises a patch that may be adhered to the skin of a patient. The patch may generally circumscribe the insertion point of the percutaneous lead and may allow a portion of the lead to extend therethrough. In this embodiment, a connector may be utilized to operatively couple the percutaneous lead with the stimulation device. Alternatively, the lead may be operatively coupled with the patch and the patch may include an adapter that operatively couples with the stimulation device. The patch may include an electrical path between the percutaneous lead and the adapter such that electrical stimulation may pass from the stimulation device through the patch and to the percutaneous lead. The lead may be coupled (either permanently or removably) to a stimulation device that was used during surgery, or another stimulation device, via a wire or other connector. The stimulation device may be placed in a sealed housing to prevent cross-contamination.

The sealed housing may comprise one or more couplers that maintain the stimulation device and stabilize the device during transportation. The housing may comprise a port that may allow the lead to be coupled with the stimulation device (or in some embodiments, the lead may be permanently attached with the stimulation device), and another port to connect a percutaneous or surface electrode (or in some embodiments, the percutaneous or surface electrode may be permanently affixed), such as a patch, as a return current path. The stimulation device may generate a signal to stimulate the nerve tissue as described in more detail herein.

In an aspect, the stimulation device may generate the signal to stimulate the nerve tissue prior to the procedure. In some embodiments, the stimulation device may additionally generate signals to stimulate the nerve tissue during the procedure (e.g., a surgical procedure), prior to surgical completion, or in lieu of a surgical procedure. Placement of a percutaneous lead may allow a surgeon to place the lead and stimulate the nerve with a stimulation device during or after the surgery, without requiring the surgeon or any other person to hold the stimulation device in place; the stimulation device may be held in an operative position without the need of human intervention. In percutaneous delivery of the stimulation, the clinician may place the electrode during the surgery. This can be accomplished in any manner. For example, the clinician or surgeon may position the percutaneous lead with an electrode at is distal end in an operative position relative to the nerve, with or without the assistance of an introducer needle. The clinician or surgeon may apply the electrical stimulation to the applicable nerve in an effort to regenerate and/or heal the nerve. In addition to or in the alternative, a clinician may position the percutaneous lead with the electrode at a distal end through ultrasound guided placement, such as when using an introducer needle. This may be done during or completely without surgery. The ultrasound guided placement may be accomplished in any manner; the present teachings are not limited to a specific method. By way of a non-limiting example, a clinician may insert an introducer needle into a patient. The clinician may use an ultrasound machine to determine the position of the introducer needle and the electrode. Once in the appropriate position, the clinician may eject the electrode and pull out the introducer needle. The electrode may then be in its operative position relative to the applicable nerve. The clinician may then apply the electrical stimulation through the electrode to the applicable nerve for the purpose of nerve regeneration and/or healing. In some embodiments a marker or identification device may be used in association with the introducer needle to help identify the location of the introducer needle through the ultra-sound.

Embodiments provide for stimulation starting at various times before a procedure, post-procedure, during a procedure or stimulation as treatment independent of procedures and for stimulation for various lengths of times. In an aspect, the stimulation may take place generally prior to a procedure for a predetermined period (e.g., i minutes, where i is a number). In at least one embodiment, the stimulation may take place for about an hour or less prior to a procedure, but may occur more than an hour before the procedure, e.g., the stimulation may comprise a short simulation of between about one and five minutes or a long stimulation of about an hour. It should be understood, however, that when disclosing the therapy times, deviation from the times disclosed are also contemplated. For example, a deviation of 20-30% may also be included as part of the disclosed times. Stimulation before a procedure (e.g., nerve transfer or nerve release) may increase the speed, quality, or amount of nerve regeneration.

In some embodiments, stimulation may occur at multiple instances leading up to a surgical procedure, during a procedure, post-procedure, and/or or stimulation as treatment independent of procedures. For example, a physician may determine a schedule for stimulation for hours, months, weeks, or days leading up to the surgical procedure or without a surgical procedure, such as an alternative to surgical procedure. The schedule may define a time period for each stimulation session (e.g., 1 minute, 5 minutes, 10 minutes, 30 minutes, 60 minutes, etc.), parameters of the stimulation signal (e.g., strength, frequency, etc.), a date/time of stimulation, or the like. In at least one embodiment, the nerve may be stimulated during multiple sessions on the day, such as the day of a procedure or according to a treatment plan. In at least one instance, prior to an operation the nerve may be stimulated for a first length of time, stimulation may be removed for a second length of time, stimulation may be applied again for a third length of time. This process of applying and resting the nerve may continue until the surgical procedure is performed and could continue for a defined period during and/or thereafter. Moreover, parameters of the stimulation signal may be different during the different stimulation sessions. In at least one example, a strength of stimulation is adjusted (e.g., increased or decreased) between stimulation sessions. In some instances, the strength of stimulation may progressively increase between sessions leading up to a procedure or may progressively decrease leading up to the procedure.

An aspect enables the onset of additional stimulation to begin in the operating room, prior to procedure completion, and additionally or alternatively continuing into a post-operative setting. In some embodiments, the stimulation may begin during the procedure and continue after the procedure without disrupting stimulation. Reduction of the delay from the completion of surgical intervention to start of stimulation, may provide further clinical benefit. This additional post procedure stimulation may be utilized in combination with pre-procedure stimulation.

It is noted that stimulation may be applied in an office, in a pre-op setting, or at another location, e.g., outside of a surgical facility or room. Moreover, placement of an electrode may be done in at any appropriate location, e.g., outside or inside of a medical facility. Application of the stimulation may likewise occur at any appropriate location. In some examples, a patient may apply stimulation at home with an appropriate stimulation device. The stimulation device may be pre-programmed with stimulation parameters, may communicate with a server to receive updated parameters, may communicate with a user device (e.g., cellular phone, laptop, desktop computer, tablet computer, etc.), or the like. The stimulation device may report to a doctor or provider regarding usage of the stimulation device.

In one example, electrical stimulation may be applied to determine a baseline level of nerve excitability before or at the start of a surgery, or before or at the start of treatment without surgery, i.e., a threshold test. During or near completion of the surgery or a treatment plan, a second test of nerve excitability may be conducted. This second test may be compared against the first. If the second test results in lower nerve excitability, prolonged stimulation may be applied after the surgery to help with nerve regeneration. This series of tests may be done with a single electrode and stimulation device or may be accomplished with two or more electrodes or stimulation devices. Further still, the initial threshold test may be done with a different stimulation and the same lead or a different stimulation device and lead (such as a stimulation device with a permanently fixed lead) or with the same stimulation device and the same lead. The application of the second test may determine if the nerve regeneration stimulation is necessary or desirable for the patient, e.g., to treat any potential nerve injury or trauma as suggested by the reduction in nerve excitability between the first and second test, or whether surgery is needed for the patient. The threshold test may comprise electrical stimulation to a nerve or nerves within a target tissue region to determine or measure the excitability of the nerve or nerves. In such situations, it may only be necessary to test nerve excitability to determine if the post procedure nerve regeneration therapy is needed prior to applying such nerve regeneration therapy. Threshold testing may be done at any time during the surgery, such as when a retractor is removed, a limb of a patient has a force applied to it, or the like. The threshold test may also be used to determine the stimulation parameters to be applied, what kind or level of stimulation dosing should be approved, or any other stimulation factors.

In another example, stimulation of a nerve may be applied before a procedure and during surgery prior to treatment or repair of nerve damage, suspected nerve damage, a risk of future nerve damage, or the like. A percutaneous lead may be placed at or near a nerve (e.g., within a range of the signal) to allow a stimulation device to generate and apply a signal to the nerve tissue. The stimulation may take place for a predetermined period of time and may be conducted at any time during surgery. For example, stimulation may occur immediately at the start of surgery and may be conducted for an hour or less. It is noted that the stimulation may be applied at different strengths, frequencies, patterns, or the like. Stimulation at time of a surgical operation may increase the speed, quality, or amount of nerve regeneration or nerve function recovery. The percutaneous lead may be anchored, held, or otherwise left in place after stimulation, before, during, and/or after a surgical operation such as without human intervention.

As described herein, the parameters of the stimulation signal for pre-procedure stimulation, during the procedure, post-procedure stimulation, or stimulation as treatment independent of procedures may be preprogrammed or may be set by a surgeon clinician. In at least one embodiment, the pulse width may be held constant (e.g., not adjusted) during the stimulation. In an aspect the stimulation signal may be applied at generally between 2-100 Hz, 15-30 Hz, or about 16-20 Hz. In some embodiments, the stimulation signal applied may be as low as 0.1 Hz or as high as 1 kHz. Still further, the stimulation signal applied by comprise randomized frequencies (within the range of between 0.1 Hz to 1 kHz), within a pre-defined range of frequencies, optimized to prevent the occurrence of pain to the patient, pseudo-random, non-constant, or a combination of any of the foregoing. The amplitude of the stimulation may be held constant or be adjusted by the surgeon. In general the stimulation amplitude will generally be between 0.1-20mA, typically between 1.0-2.0mA. Moreover, the stimulation signal may be applied for a prolonged period (e.g., an hour).

It is noted that the prolonged stimulation signal may be applied in a single dose or multiple doses, or for durations up to or more than 8 hours. For example, multiple doses may be applied through the same percutaneous lead, which may be kept in place between doses or may be removed between the doses. The multiple doses may utilize similar or different parameters. For instance, doses may progressively become shorter, longer, follow a curve, or be in any appropriate pattern. In some embodiments, the intensity of stimulation may be similar between doses or may be adjusted. Moreover the duration of a dose, whether as a single dose or repeated doses, may comprise relatively short stimulation periods (e.g., 0-5 minutes) up to relatively longer stimulation periods (e.g., up to one hour or more). In such examples, one lead may be utilized and a different stimulation device utilized depending upon the location of the patient during stimulation, i.e., pre-operative location, surgical location or post-operative location. In these embodiments, the lead may be selectively attached and removable from the stimulation device. In the alternative, one lead may be utilized and a single stimulation device utilized regardless of the location of the patient during stimulation. In these embodiments, the lead may be permanently fixed with the stimulation device or may be selectively attached and removable from the stimulation device or the lead may be permanently affixed. In yet another alternative, a different lead and different stimulation device may be utilized depending upon the location of the patient during stimulation. In these embodiments, the lead may be selectively attached and removable from the stimulation device or the lead may be permanently affixed. In such dosing examples, the electrical stimulation may be applied for a period of time, such as *k* minutes, where k is a number (e.g., 1, 2, 5, 10, 15, 60, etc.). In an example, the period may be between about 10 minutes and an hour or more.

Stimulation systems described herein may pre-operatively deliver, post-operatively deliver, or otherwise deliver (e.g., as a treatment independent of operation), for a period of time, an electrical stimulation to peripheral nerves subject to future repair. An attachment (e.g., a percutaneous electrode lead attachment such as those described in U.S. Patent Application US2014/0073985A, which is incorporated by reference herein) may be coupled with a stimulation device after a surgical procedure. It is noted that the stimulation device may be a stimulation device that is to be used or was used during the procedure. For instance, a surgeon may utilize a handheld stimulation device to apply stimulation. The handheld stimulation device may be positioned within a hermetically sealed container or within a container that is not hermetically sealed. The surgeon may place one or more implantable leads at a position where the lead may stimulate nerves that may have damage, potential for damage, or may require recovery after completion of the surgical operation. The surgeon may remove the stimulation device from the container for use in a surgical operation and may place the stimulation device in the container or a different container after the surgical operation.

It is important to note that the present disclosure contemplates providing the electrical stimulation to be preventative or as an alternative to surgery, i.e., to mitigate the impact of a nerve injury, risk of nerve injury, or suspect nerve injury in addition to during surgery. The surgeon may, for instance, place the handheld stimulation device into a sterilized container and may electrically attach one or more leads to the handheld stimulation device via ports of the container or the leads may be permanently affixed to the stimulation device. As such, the patient's nerve may be stimulated before an operation, during an operation or independent of an operation. In some instances, a surgeon may stimulate the nerve post operatively or intraoperatively via the same stimulation device utilized for the surgical operation. Once stimulation is completed, the stimulation device may be discarded or otherwise removed.

It is noted, however, that different stimulation devices may be utilized for stimulation before, during, or after a procedure. For instance, a fist stimulation device may be utilized before a procedure. This device may be reusable or disposable. During the operation, a second stimulation device may be utilized. After the operation, the surgeon may utilize the first stimulation device, the second stimulation device, or a third stimulation device to provide post operation stimulation. Moreover, stimulation devices may comprise wire connections to electrodes or may apply power via a surface coil that delivers power through the patient's skin.

As described herein, stimulation devices may be disposable or reusable. Described systems may allow a stimulation electrode to be placed intraoperatively, in close proximity to the nerve to be stimulated; allowing the lead to pass out of the tissue and attach, either permanently or selectively, to the stimulation device.

The stimulation device may provide a prolonged stimulation before an operation or as treatment without an operation. In some instances, it may additionally provide prolonged stimulation after an incision is closed or after an initial treatment period. In surgical applications, at a desired stopping time, the lead may be removed post operatively or during the operation if no post-operative stimulation is to be applied. In an aspect, this may eliminate the need for extended operating time, and may free up a surgeon or staff from holding the stimulator on the nerve for prolonged stimulation. The lead may also be left in for a period of time, to enable repeated doses of stimulation across multiple days or weeks without need for placement of another lead.

According to some embodiments, a surgeon may place a stimulation device in a container. The container may comprise a bag, box, or other container. The container may seal the stimulation device within the container to prevent or reduce the chance of contamination, provide stability, or the like. In at least one embodiment, the container may comprise one or more ports that may allow the stimulation device to be coupled with a wire or lead or may allow a lead permanently attached with the stimulation device to exit the container. The return current electrode may be attached to a side of the container that is in contact with a patient, or may be an external electrode that connects to a second port on the container. As described herein, the container may comprise a suitable material such as a plastic, vinyl, metal, or other material.

It is noted that the disclosed systems and methods are applicable for use in a wide variety of medical procedures involving peripheral nerves, whether surgical or non-surgical. By way of non-limiting example, the various aspects of the invention have application in treatment of nerve transection injuries, nerve crush injuries, suspected nerve injuries, risk of nerve injuries or function reduction, or nerve transfer procedures, including, without limitation nerve decompression procedures (such as carpal tunnel or cubital tunnel syndrome), neurolysis, nerve transfer, nerve repair (such as direct repair, autograft, allograft, or conduit), and iatrogenic injury (thermal, stretch, compression, or transection).

In at least one embodiment, an electrical stimulation system may comprise a stimulation device and an adaptor. The stimulation device may comprise a housing, a control circuitry operatively generating a stimulation signal, wherein the control circuitry is disposed within the housing, and an operative element coupled with the housing and comprising at least one electrode. The housing may be configured as a hand-held housing that is suitable for use by a surgeon/clinician using a single hand. The adaptor may be selectively attached to the operative element, and may comprise a percutaneous lead electrically coupled to the stimulation device through the at least one electrode, the percutaneous lead insertable into a patient during a subcutaneous surgery and after the subcutaneous surgery and wherein the stimulation device is capable of applying electrical stimulation during the subcutaneous surgery and after the subcutaneous surgery. The percutaneous lead may comprise strands of stainless steel wire insulated with a biocompatible material.

The control circuitry may apply the electrical stimulation before subcutaneous surgery for preventative nerve regeneration therapy, post-surgery, or for treatment without an operation. Nerve regeneration therapy may comprise stimulation to a nerve to alter recovery (e.g., improve, enhance, accelerate, etc.) of the nerve so stimulated, as an alternative to surgery, or to prevent or reduce the need for surgery. In another aspect, the control circuitry may apply the electrical stimulation for a period between 5 minutes and one hour. The electrical stimulation system may further comprise a container operatively receiving the stimulation device, wherein the container comprises at least one connection port that operatively and electrically couples the stimulation device to the percutaneous lead. In at least one embodiment, the electrical stimulation system may further comprise a splitter device, operatively coupled to the stimulation device, electrical stimulation system, and at least one other lead, wherein the splitter device receives the stimulation signal and generates generally uniform output signals to the lead and the at least one other lead. The splitter device may be disposed within the container, in a different container, or in no container. A container may comprise an attachment device that operatively attaches the container to an object. The container may additionally or alternatively comprise one or more fasteners to selectively secure the control circuitry within the container. It is noted that the at least one connection port may comprise a return port that operatively receives a return electrode of the stimulation device from within the container. The return port may operatively and electrically couple the return electrode to a return lead.

In another aspect, embodiments include a method for stimulating tissue prior to a subcutaneous surgery, and may comprise performing the subcutaneous surgery. The method may include using a stimulation device to stimulate the tissue, and placing a lead within range of a target tissue region, wherein the lead is operatively attached (either selectively or permanently) to the stimulation device, and applying a stimulation signal to the target tissue region with the lead and the stimulation device before the subcutaneous surgery. Placing the lead may comprise placing the lead percutaneously or non-percutaneously. The stimulation device may be a handheld stimulation device, or a non-handheld device. The method may include placing the stimulation device within a container before performing subcutaneous surgery.

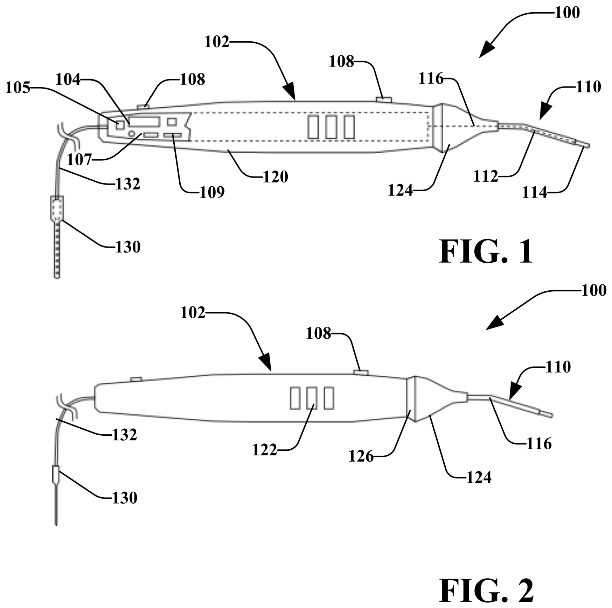

Turning now to Figs 1 and 2, there is a stimulation system 100 that may comprise a stimulation device 102 configured for locating, monitoring, and stimulating tissue and other structures throughout the body. The stimulation system 100 may be utilized for locating and identifying tissue and safeguarding against tissue and/or bone injury during surgical procedures. In another aspect, the stimulation device 102 may be utilized for percutaneously stimulating a nerve before surgery and, in some examples, during or after a surgery for a desired amount of time. In other embodiments, stimulation may be applied as a treatment program as an alternative to surgery, to prevent or reduce the need for surgery, or independent of surgery.

The stimulation device 102 may include or be coupled with one or more attachments or operative elements including, for example, a probe 110 (e.g., which may be blunt, needle-like, etc.), a cutting device, a drilling or screwing device, a pilot auger, and a fixation device. It is noted that attachments may be removable, attachable, or permanently affixed to the stimulation device 102. It is noted that while embodiments may describe use of a particular attachment (e.g., probe 110) for simplicity of explanation, the various embodiments may utilize other types of attachments.

In an exemplary embodiment, stimulation device 102 comprises control circuitry 104, disposed in a housing 120, that may apply a stimulation signal to a desired tissue region. The control circuitry 104 may be coupled to a power source, such as a battery, power mains, or the like. The control circuitry 104 may generate the stimulation signal with desired parameters, as described herein. In an aspect, a user may adjust parameters and/or control the control circuitry 104 to generate the stimulation signal via one or more user interfaces 108, which may comprise at least one of a switch, button, slide, touch screen, or the like.

For instance, a user may grasp the stimulation device 102 via the housing 120. The housing 120 may include gripping portion 122. The gripping portion 122 may comprise indents, protrusions, elastomeric material, roughened material or other features that may aid in the user grasping the stimulation device. The gripping portion 122 of the housing 120 may include an over molded portion that may comprise all or part of the length of the housing 120. In an aspect, the over molded portion may comprise a thermoplastic elastomer material. It is noted that gripping portion 122 may be removable, attached to, or integrally formed with the housing 120.

In an example, a user may position the probe 110 so that an uninsulated or stimulating portion or electrode 114 is at a desired location. This may include positioning the electrode 114 relative to a nerve or positioning the electrode 114 within a container or attachment. The user may interact with one or more of the user interfaces 108 to control delivery of stimulation signal, generated by the control circuitry 104, to the desired tissue region. The gripping portion 122 may aid in a user's efforts to hold the stimulation device 102. In an aspect, the control circuitry 104 communicates the stimulation signal to the stimulation probe 110 via a lead 112 that may travel through an insulated portion 112 of the probe to the uninsulated portion 114.

It is noted that the probe 110 may comprise one or more flexible materials (e.g., metal, plastic, etc.) so that a user may bend or otherwise manipulate the probe 110. In another aspect, the stimulation device 102 may comprise a nose cone 124 that may be flexible or rigid. An operative element (e.g., probe 110) may extend from the proximal end of the nose cone 124. The user may apply pressure to the nose cone 124 so that it moves or otherwise manipulates the probe 110. This may allow a surgeon or other user to position the uninsulated portion 114 at a desired position of a targeted tissue region. For example, the uninsulated portion 114 of the probe 110 is positioned in electrical conductive contact with at least one of muscle, nerve, or other tissue.

A flexible nose cone 124 may allow the surgeon to use either a finger or a thumb positioned on the nose cone 124 to make fine adjustments to the position of probe 110 at the targeted tissue region. The surgeon may grasp the housing 120 with the fingers and palm of the hand, and position the thumb on the nose cone 124, and with pressure applied with the thumb, cause the probe 110 to move while maintaining a steady position of the housing 120. This flexible nose cone 110 may allow for increased control of the position of the probe 110 with the movement of the surgeon's thumb (or finger, depending on how the stimulating probe is held). In another aspect, the nose cone 124 may comprise gripping components, such as ribs, indents, roughened surfaces, or the like.

It is noted that the nose cone 124 may comprise a single piece or it may comprise one or more pieces attached together. For example, nose cone 124 may comprise an inner portion that may include thermoplastic material having flexibility (e.g., LUSTRAN.RTM. ABS 348, or similar material), and an outer portion that may comprise a softer over molded portion and may be made of a thermoplastic elastomer material having flexibility (e.g., VERSAFLEX.TM. OM 3060-1 from GLS Corp). It is noted, however, that nose cone 124 may be generally rigid in at least some embodiments.

While described as a "cone" nose cone 124 may comprise a generally tapered shape or different shapes (e.g., rounded, squared, prism, conical, etc.). Moreover, in embodiments, stimulation device 102 may not comprise a nose cone 124, such that probe 110 extends directly from housing 120.

As described herein, a stimulation signal may flow from the stimulation device 102 through the lead 112 to the probe 110, which may act as an electrode. The stimulation system 100 may include one or more other electrodes, such as a return electrode, as described herein. For instance, in monopolar operation, a return electrode (or indifferent electrode) provides a return path for electrical signals passing through the tissue, and returning to the stimulation device 102. It is noted that stimulation system 100 may operate in a monopolar, bipolar or other configurations, as described here as well as elsewhere in this disclosure.

In various embodiments, the control circuitry 104 may generate the stimulation signal to operatively generate a physical motor response of a tissue (e.g., muscle, innervated muscle, nerve, etc.). The physical motor response may indicate whether the stimulation signal was delivered and/or whether a sufficient stimulation signal was delivered. For example, the motor response may include a physical motor response (e.g., twitching or contraction).

In another aspect, the stimulation device 102 may generate one or more visual or audio signals (e.g., via a speaker (not shown)), which indicate to the surgeon the status or diagnostic information. For instance, stimulation device 102 may comprise an indicator light 126. The indicator light may comprise one or more light sources, such as a light emitting diode (LED). In an aspect, the indicator light 126 may comprise a translucent (e.g., semi-translucent, fully translucent, etc.) surface that operatively shines or disperses light from an internal light source (not shown). In an aspect, the light source may generate light in one or more colors (e.g., green, yellow, blue, red, etc.), patterns (e.g., blink rate, pattern of colors, etc.), or the like. According to embodiments, the status or diagnostic information may indicate whether the stimulation signal was delivered and/or whether a sufficient stimulation signal was delivered, i.e., the requested amount of or the designated stimulation signal was delivered. For example, the status or diagnostic information may indicate that an electric signal was returned from tissue, which may indicate sufficient proximity, contact, or delivery of the stimulation signal requested via an operative element (e.g., probe 110). In another aspect, the indicator light 126 may indicate that the stimulation device 102 is on/off, producing or not producing a stimulation signal, or the like. Still further, the indicator light 126 may indicate that the stimulation signal requested from the stimulation device 102 by the user completes the electrical flow path going through the operative element (e.g., probe 110) and the targeted tissue region and back through the return electrode at a specified stimulation level equivalent to a control setting selected by the user on the stimulation device 102. The indicator light may provide, in response to determining whether the electrical stimulation signal completes the electrical flow path going through the operative element (e.g., probe 110) and the targeted tissue region and back through the return electrode in accordance with the specified stimulation level, a first indication signal for confirming delivery of the electrical stimulation signal to the targeted tissue region through the operative element and back through the return electrode completing the electrical flow path at the specified stimulation level. The indicator light 126 may also provide, in response to determining whether the electrical stimulation signal going to the operative element and the targeted tissue region and back through the return electrode is not at the specified stimulation level, a second indication signal to the at least one indicator for indicating a failure of delivery of the electrical stimulation signal to the targeted tissue region at the specified stimulation level through the operative element to the targeted tissue region and back through the return electrode. The indicator light 126 incorporated with the housing may provide reliable feedback to the surgeon/user as to the request and delivery of stimulus current.

In an example, the indicator light 126 allows the surgeon to confirm delivery of stimulus current, or more specifically, the pre-selected or desired stimulus current, to tissue. Through the use of different tones, colors, different flash rates, etc., the indicator 126 allows the surgeon to confirm that the uninsulated tip 114 is in place, the stimulation device 102/instrument is turned ON, and that stimulus current is flowing with sufficient (i.e., the desired) delivery to tissue. Thus, the surgeon has a much greater confidence that the desired stimulation amplitude is actually being delivered to the nerve, as in the case of a nerve transfer of nerve graft, a muscle contraction will not be observed since the nerve is no longer in continuity. These indicators can be checked periodically to ensure stimulation (e.g., the desired stimulation) is being delivered for the desired duration (e.g. between about 1 minute and one hour). In these embodiments, the indicator 126 is determining more than just whether the operative element is providing stimulation, it indicates whether or not the electrical stimulation signal completes the electrical flow path going through the operative element (e.g., probe 110) and the targeted tissue region and back through the return electrode at a specified stimulation level based on a control setting selected by the user on the stimulation device 102.

As another example, in use the indicator 126 may be configured to illuminate continuously in one color when the stimulation device 102 is turned on but not in contact with tissue. After contact with tissue is made, the indicator 126 may flash (i.e., blink) to indicate that stimulation is being delivered (as described above). If the stimulation has been requested, i.e., the stimulation probe has been turned on, but there is no stimulation being delivered because of a lack of continuity between the probe 110 and the return electrode 130, or an inadequate connection of the probe 110 or the return electrode 130 to the patient tissue, or the stimulation device not delivering to the tissue the desired/pre-selected stimulation signal, the indicator 126 may illuminate in a different color, and may illuminate continuously or may flash.

As described herein, the indicator 126 may comprise a ring that provides a visual indication around at least a portion, and desirably all of the circumference of the stimulation device 102 generally near the nose cone 124. A ring indicator may be an element of the gripping portion 122, or it may be an element of the flexible nose cone 124, or the ring indicator may be positioned between the gripping portion 122 and the nose cone 124. The ring may also include a reflective element to improve and focus the illumination effect of the light emitting source, e.g., one or more LEDs. The ring and the reflective element may be a single component, or more than one component. Audio feedback also makes possible the feature of assisting the surgeon with monitoring nerve integrity during surgery.

While stimulation device 102 is described as generating an indication, it is noted that various other components of the stimulation system 100 may generate all or part of the indication. For instance, the stimulation device 102 (or a separate device) may monitor delivery of the stimulation signal. The stimulation device 102 may transmit status and diagnostic information (e.g. delivered current, stimulation duration, contraction presence, or the like) to a separate device (e.g., laptop, wearable electronic device, cellular phone, tablet, computer, speakers, light source, or the like). In an aspect, the stimulation device 102 may include a communication component that may be wired or wireless. For example, the stimulation device 102 may include a wireless transmitter/receiver configured to communicate via one or more communication protocols (e.g., Wi-Fi, BLUETOOTH, NFC, etc.).

In embodiments, stimulation device 102 may comprise a hand-held stimulation device. Housing 120 may be generally tubular, hexagonal, or other elongated shape. According to an aspect, the housing 120 may be ergonomic and sterile for use in operative procedures. For instance, the stimulation device 120 may be packaged in a sealed container that may allow a surgeon to open and use the stimulation device 120 without the need for sterilization. It is noted, however, that parts of the stimulation system 100 may be sterilized, such as probe 110. In another aspect, the stimulation device 120 may comprise a single use instrument for use during surgical procedures to identify nerves and muscles, muscle attachments, contract muscles to assess the quality of surgical interventions or the need for surgical interventions, evaluate the function of nerves already identified through visual means, or provide prolonged stimulation of a nerve.

The stimulation device 120 may be sized small enough to be held and used by one hand during surgical procedures, and may be ergonomically designed for use in either the left or right hand. In an embodiment, the stimulation device 120 may have a width of about 20 millimeters to about 30 millimeters, and desirably about 25 millimeters. The length of the stimulation device 120 (not including an operative element) may be about 18 centimeters to about 22 centimeters, and desirably about 20 centimeters. An operative element (e.g., probe 110) may also include an angle or bend 116 to facilitate access to deep as well as superficial structures without the need for a large incision. As illustrated, the bend 116 may be generally downward, relative the directions shown in Figs 1 and 2. In an aspect, this may allow a surgeon to maintain a line of sight with target tissue and/or the uninsulated portion 114.

In one or more embodiments, as described here as well as elsewhere in this disclosure, an operative element may be monopolar or bipolar. For instance, probe 110 may be monopolar. A return electrode 130 may be coupled to control circuit 104 via an insulated wire 132. The return electrode 130 may comprise any of a variety of electrode types (e.g., paddle, needle, wire, or surface electrode). In another aspect, the stimulation device 102 may be bipolar and may comprise a return electrode in the probe 110 or other operative element.

User interfaces 108 may allow a user to turn ON/OFF the stimulation device 102 (or set to standby), and may allow a user to control the stimulation signal amplitude selection within a predefined range (e.g., 0.1 0.5, 2.0, and/or 20 mA). In configurations, user interface 108 may be a four or five position switch. It is noted that the user interface 108 may allow for selection and change of frequencies within a range. Before the first use of the stimulation device 102, the user interface 108 is in the OFF position and keeps the stimulation probe off. After the user interface 108 has been turned ON (e.g., by moving the switch 155 to an amplitude selection), the OFF position now corresponds to a standby condition, where no stimulation would be delivered. In one embodiment, once the stimulation device has been turned on, it cannot be turned off, it can only be returned to the standby condition and will remain operational for a predetermined time, e.g., at least about seven hours. This may allow the stimulation device 102 to be only a single use device, so it cannot be turned OFF and then used again at a later date. It is noted, however, that some embodiments may allow the user to turn off the stimulation device 102 after it has been turned on. In one example, the user interface 108 may allow for selection of "prolonged stimulation." Once prolonged stimulation has been selected, the stimulation device 102 may disable user control of certain stimulation parameters, may allow the stimulation device 102 to be turned off, or may turn off after a certain time in the prolonged stimulation mode (e.g. 1 hour).

Further still, the stimulation device 102 may shut off automatically after a pre-defined period of time, such as by way of a non-limiting example, two hours or more, one hour, thirty minutes, fifteen minutes, ten minutes, five minutes, one minute or thirty seconds. Any pre-defined time may be utilized. In these embodiments, the stimulation device 102 may include a timer 109. The timer 109 may be operatively coupled with or be integral to the control circuit 104. The time 109 may be triggered upon the stimulation device 102 being turned on by the user as previously described. For example, the user may actuate, touch or otherwise engage the user interface 108 to turn the stimulation device 102 on to an active state. The time 109 may then count down a predetermined amount of time and at the conclusion of the predetermined amount of time, the stimulation device 102 may turn off, such as without human intervention.

In some embodiments, the time 109 may continue to run for a second predetermined amount of time, which may be greater than, less than or equal to the predetermined amount of time. At the conclusion of the second predetermined amount of time, the stimulation device 102 may automatically turn back on to apply electrical stimulation or it may utilize the indicator 126 to identify to the user/surgeon that the second predetermined time has expired and the stimulation device 102 may be turned on to provide stimulation; during the second predetermined amount of time, the stimulation device 102 may not be capable of turning on. In this case, the user/surgeon or even the patient may utilize the user interface 108 to manually turn the stimulation device 102 on to apply electrical stimulation. This may allow the stimulation device 102 to provide an automatic dosing regimen. In fact, this process may be repeated for any number of stimulation cycles whereby the stimulation device 102 is automatically (without human intervention) on for the stimulation predetermined amount of time and off for a resting amount of time. Each cycle may have a different stimulation predetermined amount of time, such as a cascading amount of time, or the time may be the same as the other stimulation predetermined amount(s) of time. Similarly, the resting amount of time may be constant or may vary depending upon the dosing schedule, e.g., it may increase each successive period. Still further, the stimulation predetermined amount of time may be the same as or different from the resting amount of time. The stimulation device 102 can apply a dosing regimen utilizing cycles of the stimulation predetermined amount of time that have different stimulation patterns (or in some embodiments, the same stimulation pattern). The stimulation predetermined amount of time and the resting predetermined amount of time may comprise any appropriate period, such as for example, one or more hours, thirty minutes, twenty minutes, fifteen minutes, ten minutes, one minute or thirty seconds. The present teachings are not limited to a specific time.

In some embodiments, once the predetermined amount of time has elapsed, the stimulation device 102 may be unable to be turned back on to apply stimulation. This essentially results in the stimulation device becoming a single use device.

Still further, the stimulation device 102 may include a pulse counter 105 of any appropriate configuration. The pulse counter 105 may count the number of pulses applied by the stimulation device 102 and turn off after a predetermined number of pulses has been applied. The pulse counter 105 may be operatively coupled with or integral to the control circuit 104. Upon occurrence of the predetermined number of pulses, the stimulation device 102 may automatically turn off. The stimulation device may then use a time 109 as described above to turn on after a predetermined amount of time to apply electrical stimulation or it may utilize the indicator 126 to identify to the user/surgeon that the predetermined time has expired and the stimulation device 102 may be turned on to provide stimulation. In this case, the user/surgeon or even the patient may utilize the user interface 108 to manually turn the stimulation device 102 on to apply electrical stimulation. This may allow the stimulation device 102 to provide an automatic dosing regimen. In fact, this process may be repeated for any number of stimulation cycles whereby the stimulation device 102 is automatically (without human intervention) on for the predetermined number of pulses and off for a resting amount of time. Each cycle may have a different predetermined number of pulses, such as a cascading amount of pulses, or may have the same predetermined number of pulses. The resting amount of time may be constant or may vary depending upon the dosing schedule, e.g., it may increase or decrease each successive period. The stimulation device 102 can apply a dosing regimen utilizing cycles of the predetermined number of pulses that have different stimulation patterns (or in some embodiments, the same stimulation pattern). The predetermined number of pulses and the resting predetermined amount of time may comprise any appropriate period, such as for example, one or more hours, thirty minutes, twenty minutes, fifteen minutes, ten minutes, one minute or thirty seconds. The present teachings are not limited to a specific time and/or number of pulses.

In some embodiments, once the predetermined number of pulses has occurred, the stimulation device 102 is unable to be turned back on to apply stimulation. This essentially results in the stimulation device being a single use device.

In some embodiments, the pulse counter 105 may include a sensor or plurality of sensors that can determine the number of pulses generated from the stimulation device 102 (or more specifically from the probe 110). Still further, the pulse counter 105 may include a stimulation sensor or a plurality of stimulation sensors that sense or determine the number of pulses actually delivered through the probe 110 from the stimulation device 102 to the targeted tissues (or nerve(s)) of the patient. This stimulation sensor is able to determine if an electrical pulse emitted from the probe 110 is actually delivered to the patient. In this embodiment, therefore, the pulse counter 105 will count the total number of pulses actually delivered to the patient. If a pulse, for any reason, is not delivered to the patient, the pulse counter 105 will not count it and such pulse will not count toward the predetermined number of pulses the pulse counter 105 is counting. Further, the system may be able to compare the number of electrical pulses sent from the device against the number of pulses actually delivered to the patient. This may be utilized to determine the function of the device and/or the stimulation parameters being sent. Such sensors may comprise an electrode attached to the nerve to determine if there had been a pulse delivered to the nerve. The electrode may be configured to pick up a generally weak signal amid "background noise" in the patient.

Still further, the pulse counter 105 may include an action potential sensor 107 or plurality of action potential sensors that can determine the number of action potentials generated from the stimulation device 102 (or more specifically from the probe 110) through the nerve or nerves of the patient. The action potential sensor 107 or sensors may comprise an accelerometer, an electrode (of any appropriate configuration) or the like. In an example, the system may include one or more leads for stimulation and an action potential sensor(s) 107's comprising one or more leads for recording activity, such recording electrical activity. The action potential sensor 107 may determine generation of an action potential from the nerve or nerves being stimulated through the probe 110 by the stimulation device 102. The action potential sensor 107 is able to determine if an electrical pulse emitted from the probe 110 is actually delivered to the patient and that it generates an action potential. For example, the action potential sensor 107 may be placed on, coupled to or otherwise attached to a location on a patient whereby the action potential can be detected upon application of the stimulation. In such a case, an electrode may be located in an area proximal and/or distal to the location of the stimulation in the patient where the nerve being stimulated innervates. In an example, action potentials going to the spinal cord, going to muscle tissues, or going in other directions may be recorded. The electrode may, alone or in combination with other components (e.g., a processor and memory), determine if an action potential is generated and sends a signal to the pulse counter 105 indicating that the action potential was generated such that the pulse counter 105 can count the generation of the action potential. Still further, the action potential sensor 107 may comprise an accelerometer that may be placed on, coupled to or otherwise attached to the patient to determine if an action potential was generated through the stimulation from the stimulation device 102. The accelerometer may be useful if the stimulation being applied is strong enough to invoke a muscle contraction that the accelerometer can sense and send a signal to the pulse counter 105 indicating that an action potential was generated. Regardless of the configuration of the action potential sensor 107, the pulse counter 105 will count the total number of action potentials generated in the patient. If an action potential is, for any reason, not generated in the patient, the pulse counter 105 will not count it and such pulse will not count toward the predetermined number of action potentials the pulse counter 105 is counting. In this condition an indicator may inform the user that adequate stimulation is not being delivered and therapy paused until stimulation parameters are adjusted, or stimulation adjustment may be automatically done by the software until action potentials are observed. In some embodiments, the indicator may be triggered after a given number of not generated action potentials are not identified within a given time frame or otherwise missed (e.g., missed x consecutive, missed *i* out of the last *j,* missed x within the past y milliseconds, etc.).

It is noted that in some embodiments, one or more of the pulse counter 105, action potential sensor 107, or timer 109 may be comprised in a single device, in multiple device, within dedicated hardware and software, or as software stored within a memory and executed by a processor.

The user interfaces 108 may allow for adjustment of a stimulation signal pulse width from a predefined range (e.g., about zero to about 200 microseconds). In one embodiment, the user interface 108 may be a potentiometer to allow a slide control to increase or decrease the stimulation signal pulse width within the predefined range. The stimulation pulse may have a non-adjustable frequency in the range of about 10 Hz to about 30 Hz, and desirably about 16 Hz. In some embodiments, the stimulation pulse may comprise an adjustable frequency.

As a representative example, the stimulation pulse may have a biphasic waveform with controlled current during the cathodic (leading) phase, and net DC current less than 10 microamps, switch adjustable from about 0.1 milliamps to about 20 milliamps, and pulse durations adjustable from about zero microseconds up to about 1 millisecond.

The operative element (e.g., probe 110) exits or attaches to the housing 120 at the nose cone 124 to deliver stimulus current to the excitable tissue. The probe 110 comprises a length and a diameter of a conductive material, and is desirably fully insulated with the exception of the uninsulated portion 114, e.g. about 1.0 millimeters to about 10 millimeters, and desirably about 4 millimeters to about 6 millimeters, which is non-insulated and serves as the stimulating to allow the surgeon to deliver the stimulus.

The size of the uninsulated portion 114, (the active electrode) of the probe 110 ensures a high current density that will stimulate nearby excitable tissue. The insulation portion 112 may comprise a medical grade heat shrink.

The conductive material of the probe 110 comprises a diameter having a range between about 0.5 millimeters to about 1.5 millimeters, and may be desirably about 1.0 millimeters. The length of the operative element 110 may be about 50 millimeters to about 60 millimeters, although it is to be appreciated that the length may vary depending on the particular application. As shown, the probe 110 may include one or more bends to facilitate accurate placement of the uninsulated portion 114. In one embodiment, the conductive material of probe 110 is made of a stainless steel, solid wire, although other conductive materials may be used. Further, the probe 110 may include an anchor 116. The anchor 116 may be of any appropriate configuration. By way of a non-limiting example, the anchor 116 may comprise a bend in the probe 110 such that upon insertion of the probe 110 into tissue of a patient, or more specifically, the anchor 116 being inserted into tissue of the patient, the anchor 116 prevents an undesired withdrawal of the anchor 116 and/or probe 110.

As previously described, in monopolar operation, a return electrode 131 (or indifferent electrode), for example, provides an electrical path from the body to the stimulation device 102. The return electrode 130 may be placed on the surface of intact skin (e.g., surface electrodes as used for electrocardiogic or electromyographic monitoring during surgical procedures) or it might be needle-like and be placed in the surgical field or penetrate through intact skin or an incision.

The configuration of the stimulating medical devices that form a part of the system can vary in form and function. Various representative embodiments of illustrative medical devices will be described.

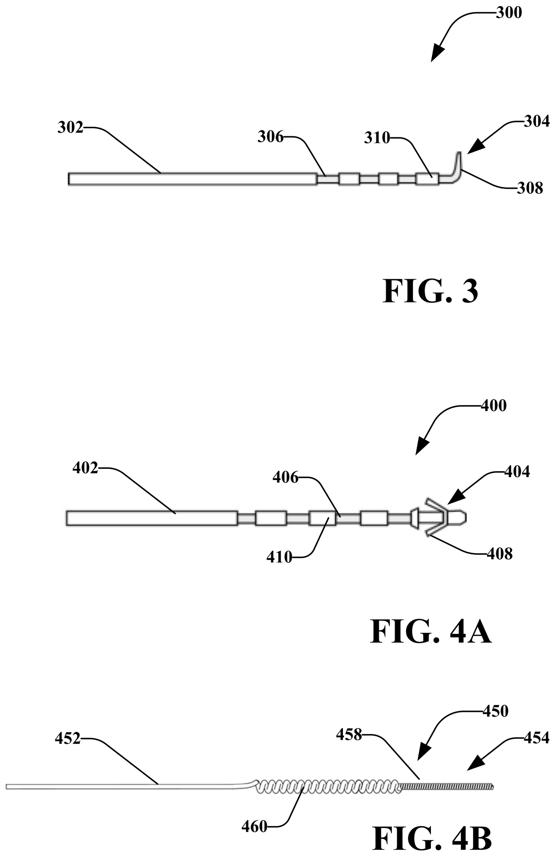

Referring now to Figs 3-4A, there are percutaneous electrodes 300 and 400 in accordance with various disclosed aspects. Percutaneous electrodes 300 and 400 may generally include an insulated body 302/402, an anchor 304/404, and one or more uninsulated portions 306/406. It is noted that percutaneous electrode 300 may comprise similar aspects as percutaneous electrode 400, unless context suggests otherwise or specific reference is made to a difference between the two. As such, while examples may refer to one of the percutaneous electrodes 300 and 400, for simplicity of explanation, the other may be utilized. Moreover, various other percutaneous electrodes may be utilized by embodiments disclosed herein.

In an example, percutaneous electrode 300 may be placed at or near a target tissue region and may be coupled with a percutaneous lead or wire, as described herein. It is noted that percutaneous electrode 300 may be positioned while an incision is open and may be left in place while the incision is closed. In at least one other embodiment, percutaneous electrode 300 may be positioned when an incision is closed or by deploying the percutaneous electrode 300.

In embodiments, percutaneous electrode 300 may comprise strands of stainless steel wire insulated with a biocompatible polymer. Each wire strand may have a diameter of approximately 34 µm and the insulated multi-strand lead wire may have a diameter of approximately 250 µm. It should be understood, however, that these dimensions and materials are merely exemplary and the present teachings are not limited to such. Any appropriate sized, shaped and configured electrode and percutaneous lead may be used. The insulated wire may be formed into a spiral or helix as has been found to accommodate high dynamic stress upon muscle flexion and extension, while simultaneously retaining low susceptibility to fatigue. The outer diameter of the percutaneous electrode 300 may be approximately 580 µm and it may be encased or filled with silicone or the like. In at least some embodiments, percutaneous electrode 300 may be made out of a different material (e.g., another metal, conducting polymer), may be insulated with another material, or may not be insulated. Further, the lead may be cylindrical or paddle-like.

Unlike surface electrodes that are applied to the surface of the patient's skin using an adhesive, percutaneous electrode 300 may be surgically implanted or otherwise inserted into select tissue. The terminal end or anchor 304 may comprise one or more tines 308 (e.g., tines 408 of percutaneous electrode 400). The anchor 304 may be insulated or uninsulated. In at least some embodiments, the anchor 304 may be inserted directly into tissue and may deliver stimulation signals to the tissue. In another aspect, the anchor 304 may generally hold the percutaneous electrode 300 in place. For instance, the one or more tines 308 may comprise a bend, curve, barb, etc., that prevents the percutaneous electrode 300 from substantially moving or unintentionally coming loose. As shown in Figs 3 and 4, disclosed embodiments may include different types of anchors 304/404. For instance, an anchor may include j tines, where j is a number. In an exemplary embodiment, a patch assembly may be utilized in conjunction with the percutaneous electrode 300. The patch assembly may comprise several layers, including an adhesive layer, an electrode layer, a reinforcement layer and a cover layer. In one embodiment, the patch assembly may include a power source for the stimulation device. Further, the patch assembly may act as a surface electrode. In one embodiment, the patch assembly may include an engagement member or members that electrically couple the stimulation device to the percutaneous electrode 300 to provide stimulation for nerve regeneration. The engagement member may comprise a snap, a magnetic male and female member capable of operable engagement, a bayonet engagement device, or any known engagement mechanisms capable of electrically coupling the stimulation device with the percutaneous electrode 300. The present disclosure contemplates any such configuration of the patch assembly.

In another aspect, an anchor may include threaded members (e.g., screws) or the like. Further still, the percutaneous electrode 300 may not include any tines or anchors. In these embodiments, the percutaneous electrode 300 may be placed near or around, i.e., generally circumscribing all of or a portion of the applicable nerve. Further, the percutaneous electrode 300 may be placed over, i.e., on top of or at the bottom of, the applicable nerve, or near, i.e., in an operative distance from the applicable nerve in any manner. The present teachings are not limited to a specific configuration. Embodiments may include a nerve cuff, a coiled lead, a straight lead, lead with a hook, lead with a tine or tines, or the like.

According to embodiments, percutaneous electrode 300 may comprise flexible materials that allow some or all of the percutaneous electrode 300 to bend or deform. In an example, the insulated portion 302 may be a lead that is generally flexible to allow removal, positioning, or other manipulation of the percutaneous electrode 300.

In embodiments, sections of the uninsulated portion 306 may be separated by insulated portions 310 (e.g., insulation portions 410 in FIG. 4A). It is noted that different sections of the uninsulated portions 306 may be electrically isolated from each other to allow for bipolar stimulation. In another aspect, the insulated portions 310 may allow for increased strength, positioning, or the like of the percutaneous electrode 300. The uninsulated portion 306 may operatively deliver a stimulation current. It is noted that the uninsulated portion 306 may be disposed anywhere along the percutaneous electrode 300. For instance, the uninsulated portion 306 may be disposed at one or more tines 308, at anchor 304, or the like.

FIG. 4B illustrates another percutaneous electrode 450 comprising an insulated body 452 and an anchor 454. The anchor 454 may comprise a twisted or braided wire. The anchor 454 may be electrically conducting and not insulated such that it may apply a stimulation signal to tissue. In an aspect, the anchor 454 may comprise a helical portion 458. In another aspect, body 452 may include twisted, braided or helical portion 460. The helical portion 458 and helical portion 460 may anchor the percutaneous electrode 450 in places. In an aspect, the percutaneous electrode 450 may allow for extended use or implantation. For example, tissue may heal around the helical portion 458 or helical portion 460. The shape of these portions allows tissue to grasp and grow in between turns or bends of the helical portion 458 and helical portion 460. The tissue growth will anchor the percutaneous electrode 450 and may prevent or reduce chances of developing infections as the tissue heals around the percutaneous electrode 450. This may allow the electrode 450 to remain in place for an extended period of time or for a short period of time. Further, the helical portion 460 may comprise a fine-coiled wire with an insulative material surrounding such.

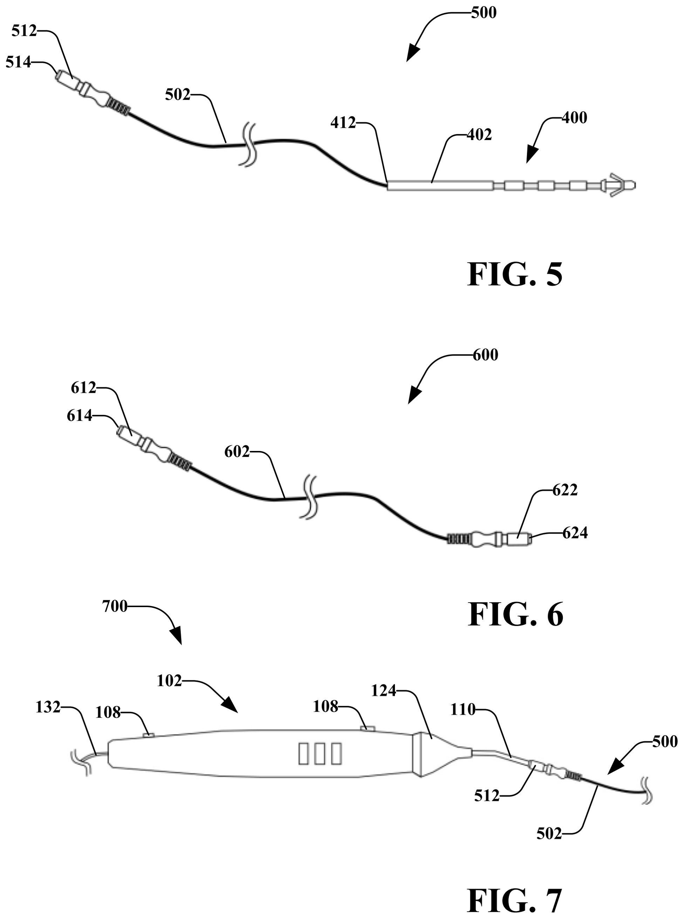

Turning now to Figs 5-7, with reference to Figs 1-4, there is adaptor 500 (which includes percutaneous electrode 400 (and may also include electrode 300 and 450) and a lead wire 502), adaptor 600 (which may be coupled to a stimulation device and/or percutaneous electrode), and stimulation system 700 (which may include stimulation device 102). It is noted that like named components of the various embodiments may comprise similar aspects, unless context suggests otherwise or a particular distinction is made.

In an embodiment, adaptor 500 may primarily include percutaneous electrode 400, wire 502 and connector 512. Wire 502 may connect terminal end 412 of the percutaneous electrode 400 with connector 512. In an aspect, wire 502 may comprise an insulated wire that is removably or irremovably attached to the percutaneous electrode 400 and/or connector 512. As described herein, the adaptor 500 may be configured to allow a stimulation probe 110 to deliver a stimulation signal below the skin of a subject patient.

In an aspect, connector 512 may include an opening 514 that may receive an operative element. As shown in FIG. 7, the opening 514 may receive the probe 110 of a stimulation device 102. The opening 514 may be tapered to maintain the probe 110 in a friction fit within the connector 512. The connector may further include other retaining features, such as a fastener (e.g., screw, clasp, threaded portions, VELCRO, magnet, etc.) to retain the connection between the connector 512 and the probe 110. It is noted that connector 512 may comprise an electrical connection disposed within the connector 512 that may operatively couple an uninsulated or stimulating portion of the probe 110 with the wire 502.

Wire 502 may extend from the connector 512. Wire 502 may be an electrical conductor in electrical connection with probe 110 when probe 110 is operatively inserted into the connector 512. It is noted that the wire 502 may be any appropriate length, such as 24 inches or a length between 12 inches and 48 inches. The lead wire may further be any appropriate gauge, such as 24 AWG wire.

Percutaneous electrode 400 may be coupled to the wire 502 at a terminal end 412. According to an embodiment, the wire 502 may be removably or irremovably coupled to the terminal end. It is noted that the wire 502 may be coupled directly to the terminal end 412 and/or may be coupled indirectly to the terminal end 412, such as through one or more other connectors (not shown). Moreover, wire 502 may be coupled to other portions of the percutaneous electrode 400. In an aspect, the connection between the wire 502 and the percutaneous electrode 400 may be insulated or uninsulated.

As shown in FIG. 6, adaptor 600 may comprise a wire 602 and one or more connectors 612/622. Each connector 612/622 may comprise an opening 614/624. In an aspect, openings 614/624 may comprise similar or different dimensions. For instance, openings 614/624 may be operatively sized and shaped to receive an operative element and/or a percutaneous electrode (e.g., percutaneous electrode 300/400). In at least one embodiment, opening 614 is operatively sized to receive an operative element, and opening 624 is operatively sized to receive a percutaneous electrode. In another aspect, connectors 612/622 may comprise elastomeric materials that may stretch, compress, or otherwise fit different sized components. Moreover, while embodiments disclose connectors 612/622 as female connectors, it is noted that one or more of connectors 612/622 may be a male connector. In another aspect, wire 602 (or 502) may comprise one or more branches or pathways such that connector 614, for example, may be electrically coupled with one or more other connectors through wire 602.

Turning to FIG. 7, and as described herein, system 700 may comprise stimulation device 102 that may be coupled with an adaptor 500 (or other described adaptors). In an aspect, a surgeon may utilize stimulation device 102 prior to or during a procedure (e.g., location of a nerve, nerve assessment, etc.). In some embodiments, the surgeon may place a percutaneous electrode or non-percutaneous electrode in a desired location prior to a surgical procedure. The surgeon may couple the electrode to the stimulation device 102 via connector 500.

As illustrated, connector 512 may be attached to the probe 110. The surgeon may utilize user interfaces 108 to select a stimulation process. For instance, the surgeon may operatively set the stimulation device 102 to deliver a prolonged stimulation to target tissue. In an aspect, prolonged stimulation may be applied prior to surgical intervention (e.g. nerve repair, nerve release, or nerve transfer). In an aspect, prolonged stimulation may be delivered to a nerve or muscle, distal to site of surgical intervention, to increase muscle viability while the nerve re-grows.

It is noted that the stimulation device 102 may comprise a preprogrammed stimulation process that may operatively generate stimulation signals for prolonged stimulation. In another aspect, user interfaces 108 may allow a user to manually program or adjust stimulation parameters, such as intensity, pattern, time, or the like.

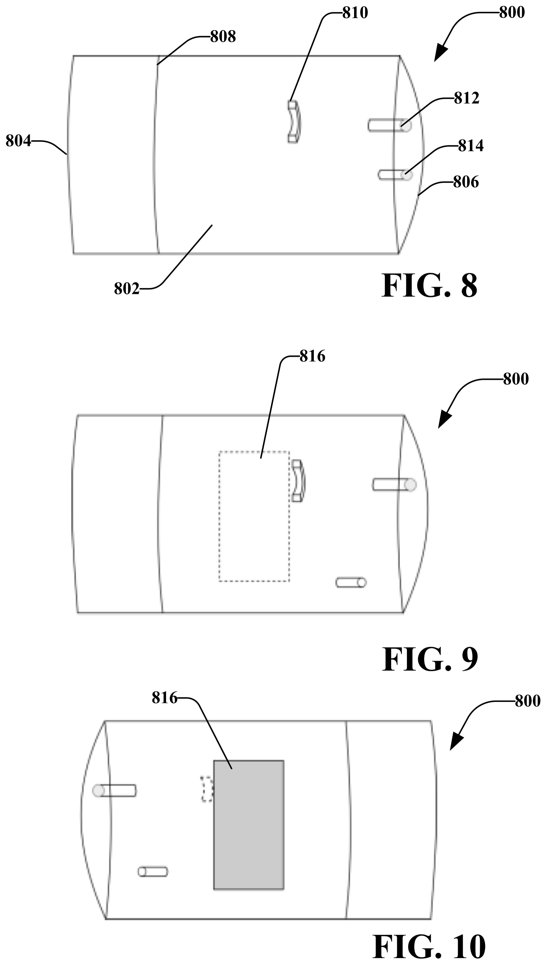

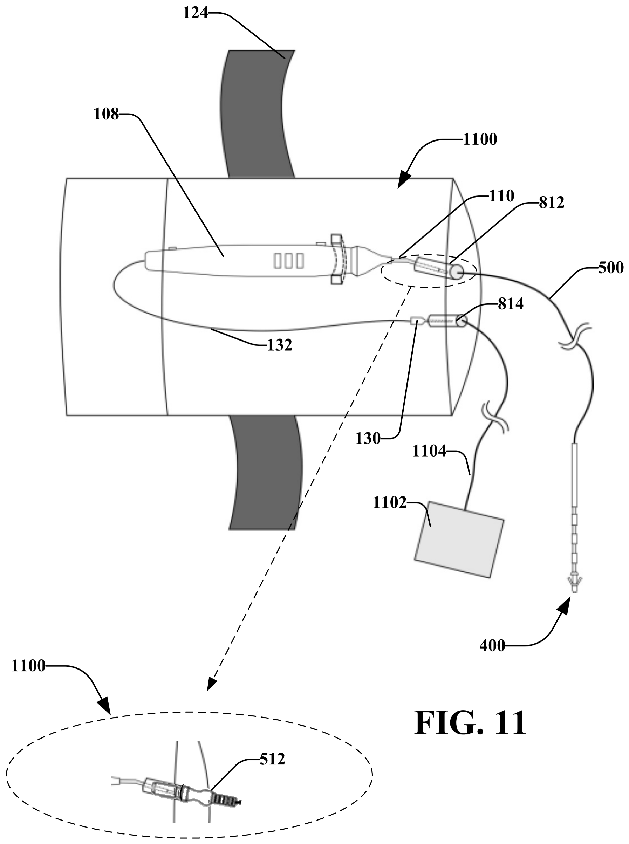

Figs 8-10 illustrate a container 800 that may operatively receive a stimulation device (e.g., stimulation device 102). FIG. 11 illustrates the stimulation device 102 disposed within the container 800 and coupled with adaptor 500.

In at least one embodiment, the stimulation device 102 may be disposed within the container 800 prior to a surgical procedure. The container 800 may be sealed to prevent contamination of the stimulation device 102 while the stimulation device 102 is within the container. In some embodiments, the stimulation device 102 may be provided within the container 800 directly from a manufacturer and may be hermetically sealed therein. As such, the stimulation device 102 is not exposed to an external environment.

The stimulation device 102 may be utilized while within the container 800 for stimulating target tissue before a surgical operation. The same stimulation device 102 may be removed from the container 800 and utilized during a surgical operation. For example, prior to entering a surgery room, the container 800 may be sterilized. Once in the room, the stimulation device 102 may be removed from the sterilized container 800 for use during a surgical operation.

In some other examples, the stimulation device 102 may be disposed in a liner (not shown) or other sealed package, and then placed within the container 800. As such, the container 800 may be discarded after prolonged stimulation and before a surgical procedure. The stimulation device 102 may then be removed from the liner for use in a surgical operation. The container 800 may comprise various materials, such as one or more of plastic, metal, or the like. In at least one embodiment, container 800 may comprise a sterilized material. Moreover, a liner may comprise various materials, such as one or more of plastic, metal, or the like.

A body 802 of the container 800 may comprise a terminal end 804 and a proximal end 806. The terminal end 804 may comprise a sealed opening 808 that may be opened when a surgeon desires to remove the stimulation device 102 from the container 800. It is noted that the sealed opening 808 may comprise an adhesive, a perforation, or the like. For instance, a user may rip or tear the perforation to expose the stimulation device 110. In some instances, the container 800 may include a pull tab that allows a user to grasp and pull the tab to open the sealed opening 808.

It is noted that various other sides or portion of the body 802 may comprise openable flaps or seals to allow the surgeon to remove the stimulation device 102 from the container 800. Moreover, a surgeon may utilize a cutting device, e.g., such as sterilized scissors or the like to open the container 800. It is noted that the container 800 may comprise indicia indicating a position at which a user is to cut to avoid harming the stimulation device 110.

According to an embodiment, container 800 may comprise one or more fasteners 810 that may fasten or hold the stimulation device 110 within the container 800. The fastener 810 may include a hook, clasp, screw, adhesive, or other fastener. For example, fastener 810 may comprise a clasp that may be sized and shaped to allow body 120 of stimulation device 102 to snap into the clasp. In an aspect, the clasp may friction fit with the body 120 to maintain the stimulation device 102 in a general position relative the container 800.

Body 802 may comprise one or more ports, such as port 812 and port 814. Port 812 and port 814 may be disposed at various locations, in an example, port 812 is disposed at a location to allow an operative element of stimulation device 102 to be inserted or otherwise coupled to port 812 when the stimulation device 102 is attached to the fastener 810. Port 814 may operatively receive return electrode 130. It is noted that port 812 and port 814 may be positioned generally proximal each other or may be positioned at other locations.

In at least some embodiments, ports 812 and 814 may allow components disposed within the container to be electrically coupled to components disposed outside of the container while maintaining the hermetic seal of the container 800, while maintaining a non-hermetic seal of the container 800 or there may be no seal maintained with the container 800. In an example, the port 812 may receive a connector (e.g., connector 512 as shown in expanded view 1100). For instance, port 812 may receive a portion of connector 512 and may operatively hold the connector 512 in place (e.g., via a friction fit, fastener, or the like).

In at least one embodiment, the port 812 (and/or 814) may comprise a connector 512 that is built into the container 800. For instance, the stimulation device 110 may be provided within the container 800 and the connector 512 may be attached to the port 812 by a user or otherwise. In another aspect, a return electrode may be coupled to another connector of port 814.

The user may connect the adaptor 500 to the port 812 from outside of the container 800 such that the container 800 remains hermetically sealed or non-hermetically sealed. In another aspect, the user may connect a return electrode 1102 to the port 814 via a wire 1104 and/or another connector. According to an aspect, this may reduce or prevent the spread of material (e.g., bodily fluids, debris etc.) from entering the container 800 and contaminating the stimulation device 110.

Body 802 may comprise other or additional components. For instance, body 802 may comprise an electrode 816 attached to the outer surface of the container. This electrode 816 may be electrically connected to the return electrode of the stimulation device through port 814. In this embodiment the integrated system would reduce the need for connection of an additional return current electrode such as 1102.

In another aspect shown in FIG. 11, body 802 may include an attachment device, such as a strap 124 that may facilitate attaching the container 800 to an object, such as a patient's limb, a belt, hospital equipment or the like. For instance, the container 800 may be attached to a patient's limb prior to a procedure for prolonged stimulation. This may allow the user to move while stimulation is applied. Various other attachment devices may be utilized, such as hook and loop materials, magnets, elastic, or the like.

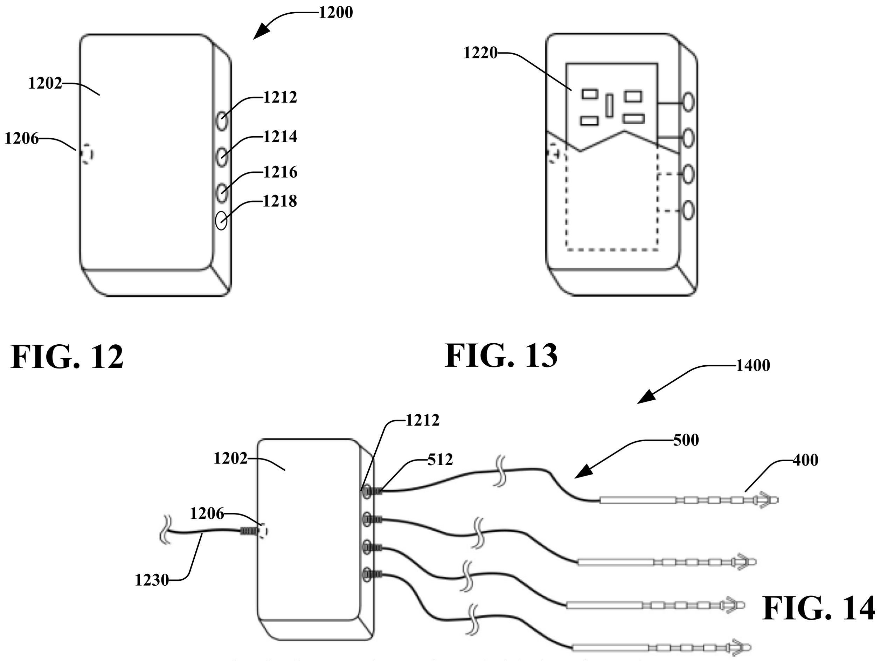

Figs 12-14 illustrate a splitter device 1200 that may operatively split a stimulation signal and/or return signal. According to embodiments, the stimulation device 1200 generally include a housing 1202 that houses circuitry 1220. The housing may include an input port 1206 and one or more output ports 1212, 1214, 1216, and 1218. It is noted that splitter device 1200 may include any number of input or output ports. Internal circuity therein may be utilized to deliver one input signal to multiple output channels, maintain current level at set output, such as for example at 1.0 or 2.0 mA.

Splitter device 1200 may receive a first wire 1230 via input port 1206. In an aspect, the first wire 1230 may be coupled to a stimulation device (not shown) that may operatively apply a stimulation signal to the input port 1206. The circuitry 1220 may split the stimulation signal to one or more of the output port(s) 1212, 1214, 1216, 1218, which may be coupled to one or more operative elements, percutaneous leads (e.g., percutaneous leads 300, 400, etc.), non-percutaneous leads, or connectors as described herein. In an example, a surgeon may place percutaneous leads 400 in different target tissue in one or more patients. The surgeon may attach the percutaneous leads 400 to the splitter device 1200 and may attach a stimulation device to the splitter device. The stimulation device 1200 may apply a prolonged stimulation signal to multiple tissue regions based via the splitter device 1200 and/or percutaneous leads 400.

As described herein, the circuitry 1220 may be an interface between a stimulation signal (received at input port 1206) and divides the input signal to one or more output signals with attached electrodes, selectively output at one or more of output port(s) 1212, 1214, 1216, and 1218. This may allow the stimulation device to utilize additional channels or contacts than otherwise available.

It is noted that the circuitry 1220 may include a power source (e.g., battery) or may receive power from the stimulation device or other external source. In an example, the circuitry may comprise an inductive circuit that operatively stores power in one or more capacitors. According to another embodiment, the input signal received at input port 1206 may supply the power. The circuitry may include the components to ensure output remains within a specified range (e.g. 2.0mA) whenever a stimulation pulse is delivered to the input port. Moreover, the circuitry 1220 may include a demodulator (e.g. to receive and decode information) and one or more switches or registers that control output to percutaneous leads 400 or other electrical contacts, or the like, In an aspect, the switches may be utilized to detect whether an electrical contact is connected to output port(s) 1212, 1214, 1216, and 1218 such that power may be selectively applied to the ports.

In embodiments the splitter device 1200, or aspects thereof, may be disposed on or within a container (e.g., container 800), or placed on the body outside the container. For instance, the housing 1202 may be disposed within layers of material in the container 800. In another aspect, the circuitry 1220 may be disposed within the material of the container 800 (e.g., the housing may comprise the material of the container). The splitter device 1200 may be sterilized prior to a surgical operation and/or after a surgical operation.

According to various embodiments, splitter device 1200 may comprise a clip, fastener, magnet, etc., that operatively attach the splitter device to a patient, clothing, hospital equipment, or the like.

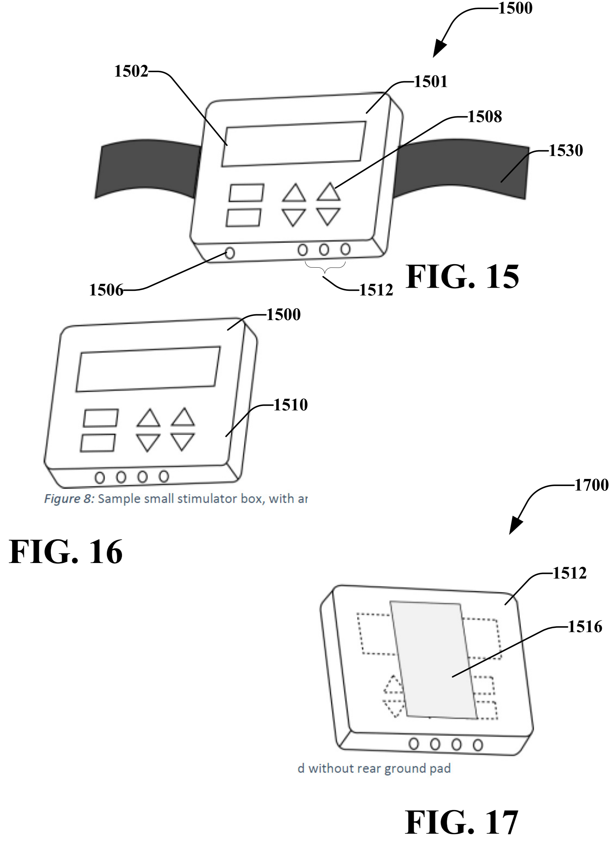

Figs 15-17 illustrate a stimulation system 1500 primarily comprising a simulation device 1501. The stimulation system 1500 may operatively control stimulation of tissue via one or more stimulating medical devices including, for example, simulation device 1501, probe 1510, percutaneous electrodes 300/400, cutting devices, drilling devices, augers, fixation devices or the like. The stimulation system 1500 may comprise an external stimulator that is selectively attachable ex-vivo a patient. The stimulation system 1500 may comprise a box that may be attached to a patient, such as by adhering to a patch assembly or other adhesive device attached to a patient, selectively attaching the stimulation device 1501 to the clothing of a patient, including a strap or necklace that a patient can wear to hold the stimulation device 1501, using hook and loop fasteners to attach the stimulation device 1501 to the patient or patient's clothes, and the like.

In an aspect, the stimulation device 1501 may comprise circuitry that operatively generates an electrical stimulation signal to be applied to a tissue. In an aspect, the stimulation device 1501 may comprise similar functionality as described with reference to other stimulation devices (e.g., stimulation device 102). Moreover, stimulation device 1501 may comprise a user interface 1508, including a display 1502. As described herein, the user interface 1508 may comprise input/output devices that operatively receive user input. A user may interact with the user interface 1508 to adjust parameters of the stimulation signal and/or receive information from the display 1502. For example, the display 1502 may indicate to a user the length of time of the current therapy has left, the time the therapy has been applied, the number of doses of therapy applied and/or number of therapies yet to be applied. By way of a non-limiting example, if the therapy has an intended duration of an hour, and twenty minutes of therapy has been applied, the display 1502 may show that there are forty minutes left on the therapy. The display 1502 may comprise an LED screen that can provide any of the information indicated in the present disclosure to the user, patient and/or clinician. Moreover, stimulation device 1501 may comprise a user interface 1508, including a display 1502 and audio feedback. In at least some embodiments, the user interface 1508 may generate an alert that is audible, visual, tactile (e.g., vibration), or combination of the above. For instance, the user interface 1508 may generate alerts to indicate that therapy is complete, a lead has moved, a lead has become disengaged, stimulation has been disrupted, an error has occurred, a warning (e.g., power supply is low), or the like. As described herein, the user interface 1508 may comprise input/output devices that operatively receive user input. The display 1502, with or without additional audio tones, may display to the user signals recorded on the one or more input ports 1506, which may be connected to other sensors or systems to record physiologic signals such as pressure, electromyograms, stretch, force, or the like. In an aspect, the stimulation device 1501 may include a communication component that may be wired or wireless. For example, the stimulation device 1501 may include a wireless transmitter/receiver configured to communicate via one or more communication protocols (e.g., Wi-Fi, BLUETOOTH, NFC, etc.).

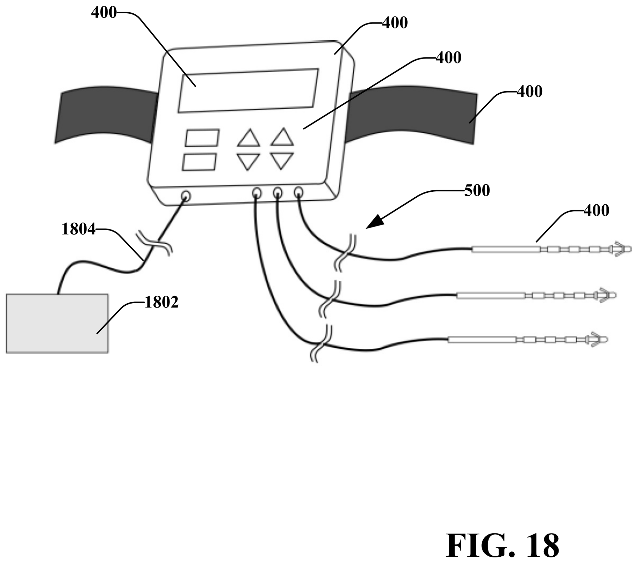

Stimulation device 1501 may include one or more output ports 1512 that may be operatively coupled with an operative element (e.g., a probe 100), one or more percutaneous leads 400 and/or connectors 500 (e.g., as shown in FIG. 18), or other components. According to one or more embodiments, the stimulation device 1501 may include one or more output ports 1506 that may be coupled to a return electrode 1802 via a wire 1804. It is noted that the return electrode 1802 may comprise a pad-type, needle, or other style electrode as described herein. In an embodiment 1700, the return electrode 1516 may be affixed to the back of the stimulation device 1512.

In embodiments, a first side 1510 of the stimulation device 1501 may comprise user interfaces 1508 as described herein. A second side 1512, which may be generally opposed to the first side 1510, may include return current electrode 1516. The second side 1512 or sides of the device may operatively receive belt 1530. Belt 1530 may be coupled to an object, such as a patient, hospital equipment, or the like. It is noted that stimulation device 1501 may be attachable to objects via other components such as clips, magnets, VELCRO, adhesives, or the like. It is further noted that stimulation device 1501 may be disposed within a container (e.g., container 800), as described with reference to Figs. 8-11. Moreover, the simulation device 1501 may apply stimulation to a target tissue region prior to a surgical operation. In some embodiments, a different stimulation device (e.g., stimulation device 110) may be utilized during the surgical operation and/or after the surgical operation.

As described herein, stimulation devices (e.g., stimulation device 102, 1501, etc.) may operatively apply prolonged stimulation to target tissue. In examples, the prolonged stimulation may be applied before a procedure is performed, during a procedure, after a procedure is preformed, or as treatment independent of surgical procedure. This stimulation may be applied in a surgery room or in a different room. If in the surgery room, tissue and other objects may be sterilize before beginning stimulation. As such, there may be no need to sterilize the stimulation device or a container prior to starting surgery. Moreover, in treatment without surgery, the stimulation may be applied in an office or at any other location.

In other examples, the stimulation may occur in a different room so that the surgical room is available for other uses. Some examples provide for disposal of the stimulation device after the pre-surgery stimulation. Other examples may provide for use of different stimulation devices before surgery and during surgery. Moreover, stimulation may be applied before surgery and not applied during or after surgery. Accordingly, an electrode may be position for pre-surgical stimulation. The electrode may then be removed during or before surgery.

In an aspect, a surgeon may selectively apply stimulation before, during or after surgery in response to assessment of nerve function in which the pre-surgical stimulation is utilized as a baseline for comparison. As an example, preoperative stimulation may be applied to the target tissue. Stimulation may be terminated and a surgical operation may be performed. During and/or after the surgical operation, the surgeon may apply stimulation to evaluate nerve function. If nerve function is below a threshold (or the threshold hold previously established), the surgeon may apply stimulation before ending the surgery, after ending the surgery, or before and after ending the surgery. In such circumstances, electrical stimulation may be applied to determine a baseline level of nerve excitability before or at the start of a surgery, or before or at the start of treatment without surgery. During or near completion of the surgery or a treatment plan, a second threshold test of nerve excitability may be conducted. This second threshold test may be compared against the first. If the second threshold test results in lower nerve excitability, prolonged stimulation may be applied after the surgery or treatment to help with nerve regeneration. The application of the second threshold test may determine if the nerve regeneration stimulation is necessary or desirable for the patient, e.g., to treat any potential nerve injury or trauma as suggested by the reduction in nerve excitability between the first and second threshold test, or whether surgery is needed for the patient. Further, the application of the second threshold test may help determine the amount of time, parameters of the electrical stimulation (e.g., amplitude of electrical stimulation, velocity, time period, pattern of stimulation, or the like), etc. applied as part of the nerve regeneration stimulation. As an example, based on severity of injury from threshold test, stimulate may be performed for longer duration or higher amplitude. For instance, if the nerve is less responsive to stimulation then a greater amplitude, such as 10mA instead of 2mA, may be applied.

The threshold test may comprise electrical stimulation to a nerve or nerves within a target tissue region to determine or measure the excitability and or conduction of the nerve or nerves. In such situations, it may only be necessary to test nerve excitability to determine if the post procedure nerve regeneration therapy is needed prior to applying such nerve regeneration therapy or to determine how the nerve regeneration therapy is to be applied. Threshold testing may be done at any time during the surgery, such as when a retractor is removed, a limb of a patient has a force applied to it, or the like.

During surgery, for instance, a surgeon may create an incision in a patient. The surgeon may utilize a stimulation device to apply a stimulation signal within the incision. Although in some embodiments, no stimulation signal may be utilized during the surgery. Instead, the stimulation may be applied only after the surgery, such as by way of a non-limiting example, during sensory nerve repair, motor or mixed nerve where a clinician does not need to or otherwise utilize a stimulator for nerve identification. It is noted that stimulation may be applied to motor or mixed nerves wherein the surgery does not utilize stimulation during the surgery. Regardless of whether stimulation was applied during surgery or after closing of the incision, the surgeon may place a percutaneous lead in or proximal target tissue. The percutaneous lead may be attached to a stimulation device. The stimulation device may apply a prolonged stimulation. In examples, the stimulation device may be the same stimulation device utilized during a procedure or may be a different stimulation device. In another aspect, the stimulation device may be disposed in a container that generally prevents contamination by or movement of the stimulation device.

As described herein, after completion of the stimulation, a surgeon or a clinician may remove the percutaneous lead. In an aspect, the percutaneous lead may be pulled out of target tissue.

In some embodiments, the stimulation described above may be done as part of an overall multi-stage treatment program. In such examples, different therapies or a pattern of the same therapies may be applied over a defined period of time as part of the nerve regeneration treatment. The overall multi-stage treatment program may extend over any appropriate amount of time. It may comprise about 5 minutes to as long as months depending upon the overall goals of the therapy, the state of the nerve to which the therapy is being applied, the healing ability of the person to whom the therapy is being applied, or any other relevant factors (e.g., excitability, conduction velocity, axon count, Schwann cell count, axon growth distance from injury site based on point of measured electrical activity, etc.) Further, the timing of the doses may comprise any appropriate time period, e.g., 1, 5, 10, 15, 30, 45 minutes, an hour, two hours or even more. The time periods between the doses may comprise any appropriate time period, e.g., 1, 5, 10, 15, 30, 45 minutes, an hour, two hours or even days, weeks or months. The timing of the doses and time periods between the dosing periods will depend on many different factors, including, without limitation, the nerve injury, amount of damage to the nerve, location of injury, patient's regenerative capability, stimulation therapy (patterns and settings of the electrical stimulation (e.g., frequency, amplitude, waveform shape, etc.)) applied to the patient, the lead utilized in the stimulation and the stimulation device utilized. It should be understood, however, that this is not an exhaustive list; any appropriate factor may be utilized in determining the appropriate timing. In at least one embodiment, recovery may be tracked and a pattern of stimulation may be adapted or altered based on patient response, such as nerve healing or regrowth identified by a physical evaluation, electrodiagnostic testing, functional testing, or the like.

An example of a multi-stage therapy may comprise providing the stimulation therapy to a nerve or nerves as described above for a first therapy time. The therapy applied could comprise any electrical stimulation therapy for nerve regeneration described above. The therapy may directly correlate to promote a healing process of one or more particular mechanisms, e.g., axon growth. This stimulation may be applied in any manner, e.g., the stimulation may be applied for a first period of time and then repeated a defined number of times over a first therapy time. During the first therapy time, the stimulation may be dosed so that the stimulation is turned off for a non-stimulation period of time and then turned back on for a second period of time with the purpose of regenerating the nerve. The dosing may be done in any manner, such as by way of a non-limiting example, as part of a random dosing (each period of stimulation is of a different time or frequency), pseudo-random (the periods of stimulation are pseudo-random and/or frequencies within a period are pseudo-random), constant, patterned (e.g., cascading or mimicking physiologic response), or any combination of the foregoing. The stimulation system 100 may be configured to apply all of the different stimulation patterns required as part of the multi-dosing program.

The multi-stage treatment program may include optimizing stimulation patterns applied to the patient to promote the healing process for one or more mechanisms of the patient. For example, the stimulation pattern applied can be optimized for angiogenesis, axon growth, cell migration (e.g., Schwann cells or macrophages), muscle and or neuromuscular junction maintenance, or any other appropriate therapy. The stimulation patterns may be optimized based on many different factors, including, without limitation, the therapy provided, the nerve injury, the amount of damage to the nerve, the tolerance of the patient, the make-up of the patient (body mass index, age, etc.), the location of the nerve to which stimulation is being applied, time point in the regenerative process, etc. The stimulation pattern may be optimized through utilization of computer modeling to determine the optimized stimulation pattern for the specific therapy and/or patient. Further, the optimized stimulation pattern may depend upon the stage of the multi-stage stimulation therapy. The optimized stimulation pattern may change depending upon whether the therapy is in an early stage or a later stage. For example, a stronger stimulation may be applied earlier in the treatment continuum than during a later stage where the nerve has begun its healing process already. Of course, the opposite may also be true, i.e., the optimized stimulation pattern may be a weaker stimulation earlier in the continuum of treatment and gets stronger during the stimulation therapy period. This may be particularly true if there is a significant injury to the nerve and only weaker stimulation can be tolerated by the patient early in the therapy. Additionally the therapy can be optimized based upon the stage of healing or regeneration. For example, early stimulation may be targeted to increase the early regenerative response (including but not limited to cell recruitment and differentiation, angiogenesis, etc.), then alter to aid in axon guidance or growth to the target, and both stages may be combined with stimulation to the innervation target to improve target viability and overall re-innervation. Any combination of the foregoing may apply.

Throughout the healing process, the stimulation patterned may be altered to reach different optimization of mechanisms for nerve healing. A pattern of mechanisms of nerve healing may be selected by the clinician determined based on prior experience, computer modeling, known therapy outcomes, etc. The stimulation system 100 may be configured to apply all of the different stimulation patterns required as part of the different optimization mechanisms contemplated herein. For example, patterns may be applied based on macrophage recruitment or Schwann cell recruitment initially followed by angiogenesis, followed by axonal guidance.

The process may further comprise delivery of biologics, drugs, other electrical stimulation therapies or other non-electrical stimulation therapies. The delivery of these additional therapies may fill gaps in the electrical stimulation treatment disclosed above to further enhance nerve regeneration and growth. For example, in addition to the electrical stimulation therapy for nerve regeneration described above, a drug treatment program may be utilized. In these embodiments, drug therapy may be applied before, contemporaneously with, or after the electrical stimulation therapy. Further, the drug therapy (or alternative therapy) may be applied as part of a dosing therapy, e.g., a period of electrical stimulation followed by a period of drug therapy, followed again by electrical stimulation, which may be again followed by drug therapy (or any alternative therapy). Any appropriate combination of electrical stimulation therapy for nerve regeneration and alternative therapy may be applied in any suitable combination. One example may include, but not be limited to, application of electrical stimulation by the stimulation system 100 for a period of time, followed by a drug therapy program. A further dose of the electrical stimulation by the stimulation system 100 may further be applied. This may also be followed by an additional dose of the drug therapy. These steps may be repeated in any order, for any number of doses, and for any appropriation duration. Further, the doses of electrical stimulation and drug therapy do not have to be consecutive, i.e., there may be a one or plurality of doses of electrical stimulation followed by a single or plurality of doses of the applicable drug therapy. Further, while the drug therapy being described as following the electrical stimulation, the present teachings are not limited to this configuration. The drug therapy dose may be applied before, contemporaneous with or after the electrical stimulation. In some embodiments, the electrical stimulation may enhance the efficacy of the drug therapy or the drug therapy may enhance the efficacy of the electrical stimulation. Further, while drug therapy has been described, any other nerve regeneration therapy may be utilized, including, without limitation, biologics, stem cell therapy, gene therapy, vitamin therapy, laser therapy, laminin-coated chitosan conduits, or any combination of the foregoing. The electrical stimulation for nerve regeneration applied may be the primary treatment or may be an ancillary or secondary treatment with any of the other foregoing treatments. Each of these treatments may be utilized a part of a dosing program, such as with immunosuppression drugs (e.g., tacrolimus also known as FK506). The present teachings are not limited to just those iterations described herein, which for the sake of brevity are not all described but are contemplated by this teaching.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

In the following different additional aspects of the invention will be described.

According to a first additional aspect of the invention an electrical stimulation system is provided, comprising: a stimulation device comprising a housing; control circuitry operatively generating a stimulation signal, wherein the control circuitry is disposed within the housing; a container housing the stimulation device, the container comprising at least one port, and wherein the container is sealed, wherein the container prevents contamination of the stimulation device such that the stimulation device may be used during at least two occasions selected from before subcutaneous surgery, during subcutaneous surgery, or after subcutaneous surgery.

In the electrical stimulation system of the first additional aspect the port may operatively couple a lead to the stimulation device :

In the electrical stimulation system of the first additional aspect the stimulation device may operatively generate a first stimulation signal for application to a target tissue region before the subcutaneous surgery and a second stimulation signal after the subcutaneous surgery for stimulation of the target tissue region after the subcutaneous surgery.

In the electrical stimulation system of the first additional aspect the stimulation device may operatively generates a third stimulation signal for stimulation of the target tissue region during the subcutaneous surgery.

The stimulation device may terminate generation of the first stimulation signal before the subcutaneous surgery begins and not generate another stimulation signal until after the subcutaneous surgery ends.

Alternatively, the stimulation device may generate the first stimulation signal between generally ten minutes and generally 15 minutes.

Alternatively, the stimulation device may generate the second stimulation signal between generally ten minutes and generally 15 minutes.

The stimulation device may generate the third stimulation signal between generally ten minutes and generally 15 minutes.

The first stimulation signal may comprise different attributes as the second stimulation signal.

In the electrical stimulation system of the first additional aspect the stimulation device may further comprise a pulse counter operatively counting the number of pulses generated by the control circuitry.

In the electrical stimulation system of the first additional aspect the stimulation device may further comprise a timer operatively measuring passage of time during which the control circuitry is generating a stimulation signal.

In the electrical stimulation system of the first additional aspect the stimulation device may further comprise an action potential sensors operatively sensing invoked action potential through the target tissue region.

According to a second additional aspect a method for stimulating tissue is provided, comprising: providing a stimulation device and a container housing the stimulation device; placing a lead within range of a target tissue region, wherein the lead is operatively attached to the stimulation device; applying a first stimulation signal to the target tissue region with the lead and the stimulation device; terminating the applying the first stimulation signal; performing, after terminating the application of the first stimulation signal, a subcutaneous surgery; and applying, after terminating the application of the first stimulation signal, a second stimulation signal to the target tissue region.

The method according to the second additional aspect may further comprise applying the second stimulation signal after the subcutaneous surgery.

The method according to the second additional aspect may further comprise applying a third stimulation signal during the subcutaneous surgery.

The method according to the second additional aspect may further comprise applying the second stimulation signal during the subcutaneous surgery.

The method according to the second additional aspect may further comprise applying the first stimulation signal comprises applying the first stimulation signal for at least generally ten minutes.

In the method according to the second additional aspect, applying the second stimulation signal may comprise applying the second stimulation signal for at least generally ten minutes.

According to a third additional aspect an electrical stimulation system is provided, comprising: a stimulation device comprising a housing; control circuitry operatively generating a stimulation signal, wherein the control circuitry is disposed within the housing; wherein the stimulation device operatively generates a first stimulation signal for application to a target tissue region to promote a healing process of a first mechanism and wherein the stimulation device operatively generates a second stimulation signal for stimulation of the target tissue region to promote a healing process of a second mechanism.

In the electrical stimulation system of the third additional aspect the first stimulation signal may be applied for a first dosing period, the first dosing period being between generally ten minutes and generally 15 minutes.

In the electrical stimulation system of the third additional aspect the first stimulation signal is applied for a plurality of dosing periods, wherein each of the dosing periods are for an equivalent amount of time.

In the electrical stimulation system of the third additional aspect the first stimulation signal may be applied for a plurality of dosing periods, wherein each of the dosing periods are for a different amount of time.

In the electrical stimulation system of the third additional aspect the second stimulation signal may be applied between generally ten minutes and generally 15 minutes.

According to a fourth additional aspect an electrical stimulation system is provided, comprising: a stimulation device comprising; a hand-held housing configured to be held in a single had of a user; control circuitry operatively generating a stimulation signal, wherein the control circuitry is disposed within the hand-held housing; wherein the stimulation device operatively generates a first stimulation signal for application to a target tissue region to promote a healing process of a first mechanism.

In the electrical stimulation system of the fourth additional aspect the stimulation device may operatively generate a second stimulation signal for stimulation of the target tissue region to promote a healing process of a second mechanism.

## Claims

1. An electrical stimulation device, comprising:
a housing;
control circuitry operatively generating a stimulation signal, wherein the control circuitry is disposed within the housing;
a percutaneous electrode connected with the stimulation device, wherein the percutaneous electrode is configured to be placed at or near a target nerve in a target tissue region that is proximal to a site of a nerve injury;
wherein the stimulation device operatively generates an electrical stimulation signal having a frequency of 0.1 Hz to 1 kHz and delivers a stimulus current to excitable tissue of the target nerve of the target tissue region to promote a healing process of the nerve injury for a therapy duration comprising 10 minutes or more but less than 30 minutes.

2. The electrical stimulation device of claim 1, wherein first stimulation signal is applied for a dosing period, the dosing period being between generally ten minutes and generally 15 minutes.

3. The electrical stimulation device of claim 1, wherein the 1 stimulation signal is applied for a plurality of dosing periods, wherein each of the dosing periods are for an equivalent amount of time.

4. The electrical stimulation device of claim 1, wherein the stimulation signal is applied for a plurality of dosing periods, wherein each of the dosing periods are for a different amount of time.

5. The electrical stimulation device of claim 1, wherein the control circuitry operatively generates a second stimulation signal for stimulation of the target tissue region during a subcutaneous surgery.

6. The electrical stimulation device of claim 1, wherein the control circuitry terminates generation of the stimulation signal before a subcutaneous surgery begins and does not generate another stimulation signal until after the subcutaneous surgery ends.

7. The electrical stimulation device of claim 1 further comprising a pulse counter operatively counting the number of pulses generated by the control circuitry.

8. The electrical stimulation device of claim 1 further comprising a timer operatively measuring passage of time during which the control circuitry is generating a stimulation signal.

9. The electrical stimulation device of claim 1 further comprising an action potential sensors operatively sensing invoked action potential through the target tissue region.

10. The electrical stimulation device of claim 1, wherein the stimulation signal comprises a biphasic waveform with controlled current during a cathodic phase.

11. The electrical stimulation device of claim 1, further comprising a plurality of channels to deliver the stimulation signal.

12. The electrical stimulation device of claim 1 further comprising a plurality of contacts to deliver the stimulation signal.

13. The electrical stimulation device of claim 1, wherein the stimulation signal applied has a frequency of 16 Hz to 20 Hz.

14. The electrical stimulation device of claim 1, wherein the stimulation signal applied has an amplitude between 0.1 mA and 20 mA.

15. The electrical stimulation device of claim 1, wherein the stimulation signal applied has an amplitude between 1 mA and 2 mA.
